# EUROPÄISCHE PATENTSCHRIFT

(11) **EP 2 155 728 B1**
(45) Veröffentlichungstag und Bekanntmachung des Hinweises auf die Patenterteilung: **29.07.2015**
(21) Anmeldenummer: 08748994.4
(22) Anmeldetag: 18.04.2008
(51) Int. Cl.: C07D 403/12, C07D 413/12, A61K 31/4245, A61K 31/4196

(54) **HETEROCYCLISCHE INDAZOLDERIVATE**
HETEROCYCLIC INDAZOLE DERIVATIVES
DÉRIVÉS D'INDAZOLE HÉTÉROCYCLIQUES

(30) Priorität: 14.05.2007 DE 102007022565
(43) Veröffentlichungstag der Anmeldung: 24.02.2010
(73) Patentinhaber: Merck Patent GmbH, 64293 Darmstadt (DE)
(72) Erfinder: KLEIN, Markus, 64291 Darmstadt (DE); BEIER, Norbert, 64354 Reinheim (DE)
(86) Internationale Anmeldenummer: PCT/EP2008/003143
(87) Internationale Veröffentlichungsnummer: WO 2008/138448

(56) Entgegenhaltungen:
- WO-A-02/10137
- WO-A-2005/051942
- WO-A-2005/123688
- WO-A-2008/086854

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der zellvolumenregulierten humanen Kinase h-sgk (human serum and glucocorticoid dependent kinase oder SGK) eine Rolle spielt, ferner pharmazeutische Zusammensetzungen, die diese Verbindungen enthalten, sowie die Verwendung der Verbindungen zur Behandlung SGKbedingter Krankheiten.

Die SGK mit den Isoformen SGK-1, SGK-2 und SGK-3 sind eine Serin/Threonin-Proteinkinase Familie (WO 02/17893).

Die erfindungsgemäßen Verbindungen sind vorzugsweise selektive Inhibitoren der SGK-1. Ferner können sie Inhibitoren der SGK-2 und/oder SGK-3 sein.

Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Tumorzellen und Tumormetastasen hemmen und sind deshalb für die Tumortherapie geeignet.

Die erfindungsgemäßen Verbindungen finden auch Verwendung bei der Behandlung von peptischen Ulcera, insbesondere bei Formen, die durch Stress ausgelöst werden.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung und Stress entgegen und steigern somit die Lebenserwartung und die Fitness im Alter.

Die erfindungsgemäßen Verbindungen finden ferner Verwendung bei der Behandlung von Tinitus.

Die Identifikation von kleinen Verbindungen, die die Signaltransduktion der SGK spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften der SGK.

Die erfindungsgemäßen Verbindungen zeigen weiterhin Aktivität auf andere Kinasen wie Aurora-B, MAPK2, MSK1, PRK2, DYRK1, CHK2, GSK3-beta, PKB (AKT), ROCKII oder S6K1.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

### STAND DER TECHNIK

In der WO 00/62781 ist die Verwendung von Arzneimitteln enthaltend Hemmstoffe der zellvolumenregulierten humanen Kinase H-SGK beschrieben.

Andere heterocyclische Indazolderivate zur Behandlung von Diabetes und/oder Krebserkrankungen kennt man aus WO 2006/044860 und WO 2005056550.

In US 2005090529 sind andere Indazolderivate zur Behandlung von diabetischer Retinopathie offenbart.

Andere Indazolderivate sind als Cytokinininhibitoren in WO2005023761 beschrieben.

In US 2006004043 und US 2005107386 sind wiederum andere Indazolderivate u.a. zur Krebsbekämpfung beschrieben.

Andere Indazolderivate zur Behandlung von Tumoren sind in WO 2005000813 offenbart, solche zur Behandlung von cardiovasculärer Erkrankungen in WO 2004060318.

Andere heterocyclische Verbindungen zur Behandlung von Tumoren kennt man aus WO2004052280.

Ferner sind andere Heterocyclen zur Behandlung psychotischer Erkrankungen in EP 328200 offenbart.

Andere Indazolderivate sind als Proteinkinase-Inhibitoren in der WO 03/064397 beschrieben.

In Bioorganic & Medicinal Chemistry Letters 13 (2003) 3059-3062 beschreibt J. Witherington et al. die Herstellung anderer Indazolderivate. Andere Indazolderivate sind als Kinaseinhibitoren in WO 2003097610 beschrieben.

Andere Indazolderivate sind als GSK-3-Inhibitoren in WO 2003051847 offenbart.

Die Herstellung von Indazolverbindungen, die als Rho-Kinase-Inhibitoren wirken, kennt man aus WO 2005035506.

Die Herstellung von Aminoindazolen, die als Protein Tau Phosphorylierungs-Inhibitoren wirken, ist in WO 2004062662, FR 2848554, WO 2004022544 und FR 2844267 offenbart.

Die Verwendung von Kinase-Inhibitoren in der antiinfektiösen Therapie ist von C.Doerig in Cell. Mol. Biol. Lett. Vol.8, No. 2A, 2003, 524-525 beschrieben.

Die Verwendung von Kinase-Inhibitoren bei Fettsucht ist von N.Perrotti in J. Biol. Chem. 2001, März 23; 276(12):9406-9412 beschrieben.

In nachstehenden Literaturstellen wird die Verwendung von SGK-Hemmern bei der Behandlung von Krankheiten nahegelegt und/oder beschrieben:
1: Chung EJ, Sung YK, Farooq M, Kim Y, Im S, Tak WY, Hwang YJ, Kim YI, Han HS, Kim JC, Kim MK. Gene expression profile analysis in human hepatocellular carcinoma by cDNA microarray. Mol Cells. 2002;14:382-7.
2: Brickley DR, Mikosz CA, Hagan CR, Conzen SD. Ubiquitin modification of serum and glucocorticoid-induced protein kinase-1(SGK-1). J Biol Chem. 2002;277:43064-70.
3: Fillon S, Klingel K, Warntges S, Sauter M, Gabrysch S, Pestel S, Tanneur V, Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002;12:47-54.
4: Brunet A, Park J, Tran H, Hu LS, Hemmings BA, Greenberg ME. Protein kinase SGK mediates survival signals by phosphorylating the forkhead transcription factor FKHRL1 (FOXO3a). Mol Cell Biol 2001;21:952-65
5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J Biol Chem. 2001;276:16649-54.
6: Zuo Z, Urban G, Scammell JG, Dean NM, McLean TK, Aragon I, Honkanen RE. Ser/Thr protein phosphatase type 5 (PP5) is a negative regulator of glucocorticoid receptor-mediated growth arrest. Biochemistry. 1999;38:8849-57.
7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J Biol Chem. 1999;274:7253-63.
8: M. Hertweck, C. Göbel, R. Baumeister: C.elegans SGK-1 is the critical component in the Akt/PKB Kinase complex to control stress response and life span. Developmental Cell, Vol. 6, 577-588, April, 2004.

### ZUSAMMENFASSUNG DER ERFINDUNG

Die Erfindung betrifft einzelne Verbindungen gemäß Anspruch 1 umfaßt von der Formel I 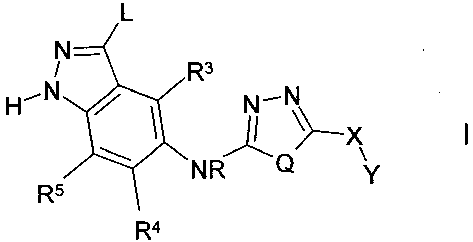 worin
- L: R¹, R² oder -(X)ₘR³,
- Q: O, NH oder N-NH₂,
- R: H oder A,
- X: CR⁷R⁸, CR⁷R⁸CR⁹R¹⁰, CR⁷R⁸C(OR⁹)R¹⁰, NR⁷, O, NR⁶CR⁷R⁸, CR⁷R⁸NR⁹, OCR⁷R⁸, OCR⁷R⁸CR⁹R¹⁰, CR⁷R⁸O, CR⁷R⁸CR⁹R¹⁰, NR⁶CR⁷R⁸CR⁹R¹⁰, CR⁷R⁸SO₂, NR⁷CONR⁸, NR⁷CONR⁸CR⁹R¹⁰, COCR⁷R⁸, CONR⁷, CONR⁷CR⁸R⁹, CONHNH, NR⁷CR⁸R⁹CONR¹⁰, NR⁷CO oder NR⁷COCR⁸R⁹,
- Y: H, A, Ar oder Het,
- R¹: CR⁹=CR⁹R¹⁰ oder CR¹²=CR¹³R¹⁴,
- R²: C≡CR¹² oder C=C-Het,
- R³, R⁴, R⁵: jeweils unabhängig voneinander H, A, Hal, OH, OA, -[C(R⁷)₂]ₙAr, -[C(R⁷)₂]ₙHet, OAr, OHet, SH, SA, SAr, SHet, NH₂, NHA, NAA', NHAr, N(Ar)₂, NHHet, N(Het)₂, NAAr, NAHet, SOA, SOAr, SOHet, SO₂A, SO₂Ar, SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr, COHet, SO₃H, SO₂NH₂, SO₂NHAr, SO₂N(Ar)₂, SO₂NHHet oder SO₂N(Het)₂,
- R⁶, R⁷, R⁸, R⁹, R¹⁰: jeweils unabhängig voneinander H oder A,
- R¹¹: Alkyl mit 1-6 C-Atomen, worin 1-5 H-Atome durch F ersetzt sein können,
- R¹², R¹³, R¹⁴: jeweils unabhängig voneinander H oder Ar,
- A, A': jeweils unabhängig voneinander unsubstituiertes oder ein-, zwei- oder dreifach durch R³, =S, =NR⁷ und/oder =O (Carbonylsauerstoff) substituiertes Alkyl mit 1-10 C-Atomen, worin eine, zwei oder drei CH₂-Gruppen durch O, S, SO, SO₂, NH, NR¹¹ und/oder durch -CH=CH-Gruppen und/oder auch 1-7 H-Atome durch F und/oder Cl ersetzt sein können, oder cyclisches Alkyl mit 3-7 C-Atomen,
- Ar: unsubstituiertes oder ein-, zwei-, drei-oder vierfach durch A, Hal, OH, OA, Ar', OAr', Het, OHet, SH, SA, SAr', SHet, NH₂, NHA, NAA', NHAr', N(Ar')₂, NHHet, N(Het)₂, NAAr', NAHet, SOA, SOAr', SOHet, SO₂A, SO₂Ar', SO₂Het, NO₂, CN, COOH, COOA, CONH₂, CONHA, CONA₂, NHCOA, NACOA, NHCONH₂, NHCONHA, NHCONA₂. NHSO₂A, NASO₂A, CHO, COA, COAr', COHet, SO₃H, SO₂NH₂, SO₂NHAr', SO₂N(Ar')₂, SO₂NHHet und/oder SO₂N(Het)₂ substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der ein-, zwei- oder dreifach durch A, Hal, OH, OA, Ar, OAr, Het', OHet', SH, SA, SAr', SHet', NH₂, NHA, NAA', NHAr', N(Ar')₂, NHHet', N(Het')₂, NAAr', NAHet', SOA, SOAr', SOHet', SO₂A, SO₂Ar', SO₂Het', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHet', SO₃H, SO₂NH₂, SO₂NHAr', SO₂N(Ar')₂, SO₂NHHet' oder SO₂N(Het')₂, =S, =NR⁷ und/oder =O (Carbonylsauerstoff) substituiert sein kann,
- Ar': unsubstituiertes oder ein-, zwei-, drei-oder vierfach durch A, Hal, OH, OA, OPhenyl, SH, SA, NH₂, NHA, NAA', NHPhenyl, SOA, SOPhenyl, SO₂A, SO₂Phenyl, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COPhenyl, SO₃H, SO₂NH₂, SO₂NHPhenyl und/oder SO₂N(Phenyl)₂ substituiertes Phenyl,
- Het': einen ein- oder zweikernigen gesättigten, ungesättigten oder aromatischen Heterocyclus mit 1 bis 4 N-, O- und/oder S-Atomen, der ein-, zwei- oder dreifach durch A, Hal, OH, OA, NH₂, NHA, NAA', SOA, SOAr', SO₂A, SO₂Ar', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', SO₃H, SO₂NH₂, SO₂NHAr', SO₂N(Ar')₂, =S, =NR⁷ und/oder =O (Carbonylsauerstoff) substituiert sein kann,
- Hal: F, Cl, Br oder I,
- m: 0, 1, 2 oder 3,
- n: 0, 1 oder 2,bedeuten,
sowie ihre pharmazeutisch verwendbaren Salze, Solvate und Stereoisomere, einschließlich deren Mischungen in allen Verhältnissen.

Gegenstand der Erfindung sind die beanspruchten Verbindungen der Formel I und ihre Salze sowie ein Verfahren zur Herstellung von Verbindungen der Formel I nach Anspruch 1 sowie ihrer pharmazeutisch verwendbaren Solvate, Salze und Stereoisomere, dadurch gekennzeichnet, daß man sie aus einem ihrer funktionellen Derivate durch Behandeln mit einem solvolysierenden oder hydrogenolysierenden Mittel in Freiheit setzt, indem man eine konventionelle Aminoschutzgruppe durch Behandeln mit einem solvolysierenden oder hydrogenolysierenden Mittel durch Wasserstoff ersetzt oder
eine durch eine konventionelle Schutzgruppe geschützte Aminogruppe in Freiheit setzt,
und/oder eine Base oder Säure der Formel I in eines ihrer Salze umwandelt.

Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen, z.B. Gemische zweier Diastereomerer oder Enantiomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen, insbesondere liegen die erfindungsgemäßen Verbindungen als Racemat vor.

Für alle Reste, die mehrfach auftreten, gilt, daß deren Bedeutungen unabhängig voneinander sind.

Vor- und nachstehend haben die Reste bzw. Parameter L, Q, R, X, Y, R³, R⁴ und R⁵ die bei der Formel I angegebenen Bedeutungen, falls nicht ausdrücklich etwas anderes angegeben ist.

A bedeutet Alkyl, ist unverzweigt (linear) öder verzweigt, und hat 1, 2, 3, 4, 5, 6, 7, 8, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1- , 2- , 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorpheny(, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylamino-phenyl, 2,3-Diaminophenyl, 2,3,4-, 2,3,5-, 2,3,6-, 2,4,6- oder 3,4,5-Tri-chlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise unsubstituiertes oder ein-, zwei-, drei- oder vierfach durch A, Hal, OH und/oder OA substituiertes Phenyl, wie z.B. o-, m- oder p-Methoxyphenyl, o-, m- oder p-Hydroxyphenyl, o-, m- oder p-Fluorphenyl, o-, m- oder p-Chlorphenyl, 2,3-, 2,4-, 2,5-, 2,6-, 3,4- oder 3,5-Dichlorphenyl, 2,3-, 2,4-, 2,5-, 2,6-, 3,4-, 3,5-Difluorphenyl oder 3-Chlor-4-fluor-phenyl.

Ar' bedeutet vorzugsweise Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyi, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4-oder-5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet vorzugsweise einen einkernigen aromatischen Heterocyclus mit 1 bis 4 N-, O- und/oder S-Atomen.

Het bedeutet besonders bevorzugt 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl.

Het bedeutet ganz besonders bevorzugt 2- oder 3-Furyl, 2- oder 3-Thienyl.

Het' bedeutet vorzugsweise einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann.

In einer weiteren Ausführungsform bedeutet Het' besonders bevorzugt unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl.

L bedeutet vorzugsweise R¹ oder -(X)ₘR³, besonders bevorzugt R¹, H, Hal, A, -[C(R⁷)₂]ₙHet oder NR⁷R⁸, ganz besonders bevorzugt H, Cl, F oder NH₂.

X bedeutet vorzugsweise CR⁷R⁸, CR⁷R⁸CR⁹R¹⁰, CR⁷R⁸C(OR⁹)R¹⁰, NR⁷, O, NR⁶CR⁷R⁸, CR⁷R⁸NR⁹, OCR⁷R⁸, OCR⁷R⁸CR⁹R¹⁰, CR⁷R⁸O, CR⁷R⁸CR⁹R¹⁰, NR⁶CR⁷R⁸CR⁹R¹⁰, CR⁷R⁸SO₂, CONHNH oder NR⁷COCR⁸R⁹.

X bedeutet besonders bevorzugt CH₂, CH₂CH₂, CH(CH₃)CH₂, CH₂CH(CH₃), CH₂CH(OH), NH, O, NHCH₂, NHCH(CH₃), CH(CH₃)NH, CH₂NH, OCH₂, OCH₂CH₂, CH(CH₃)O, CH₂O, CH₂CH₂O, NHCH₂CH₂, NHCH(CH₃)CH₂, CH₂SO₂ oder CONHNH.

R bedeutet vorzugsweise H.

Q bedeutet O, NH oder N-NH₂, besonders bevorzugt O.

R¹ bedeutet vorzugsweise CH=CH₂, CH=CHCH₃ oder CH=CH-Phenyl.

R² bedeutet vorzugsweise C=CH oder C=C-Het.

R³ bedeutet vorzugsweise H, Hal, A oder SAr.

R⁴, R⁵ bedeuten vorzugsweise H.

R⁶, R⁷, R⁸, R⁹, R¹⁰ bedeuten vorzugsweise, jeweils unabhängig voneinander H oder ^{R}".

R⁶, R⁷, R⁸, R⁹, R¹⁰ bedeuten besonders bevorzugt, jeweils unabhängig voneinander H oder CH₃.

m bedeutet vorzugsweise 0 oder 1, besonders bevorzugt 0.

Die Verbindungen der Formel I können ein oder mehrere chirale Zentren besitzen und daher in verschiedenen stereoisomeren Formen vorkommen. Die Formel I umschließt alle diese Formen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Die eingesetzten Isothiocyanate (z.B. Beispiel 1, Methode 1, Schritt1) können nach Standard-Methoden aus den entsprechenden BOCgeschützten Aminen hergestellt werden, z.B. durch Umsetzung mit Thiophosgen.

Beispiele für Isothiocyanate, die als Edukte für die erfindungsgemäßen Verbindungen eingesetzt werden:

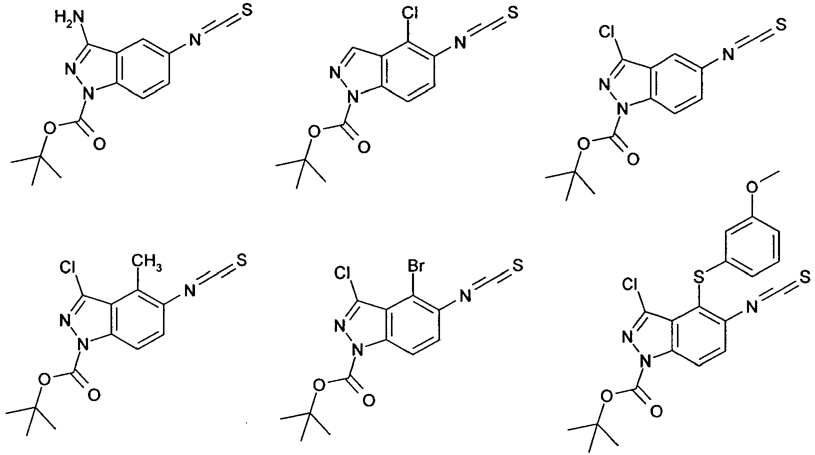

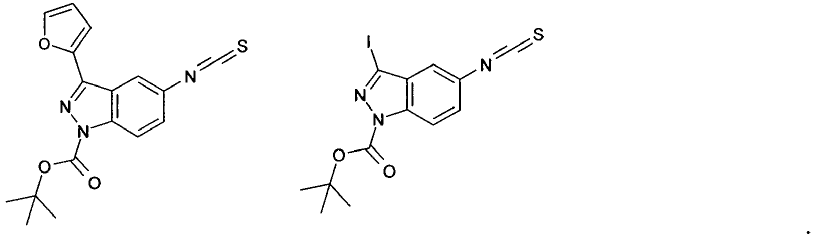

Die eingesetzten Hydrazide (z.B. Beispiel 1, Methode 1, Schritt1) können nach Standard-Methoden aus den entsprechenden Estern durch Umsetzung mit Hydrazin erhalten werden.

Beispiele für Hydrazide, die als Edukte für die erfindungsgemäßen Verbindungen eingesetzt werden:

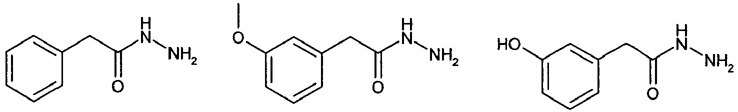

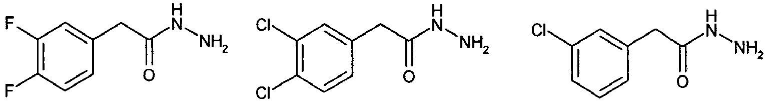

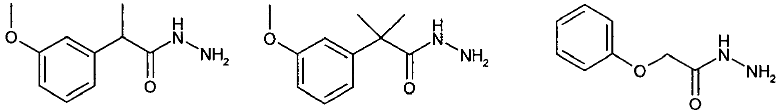

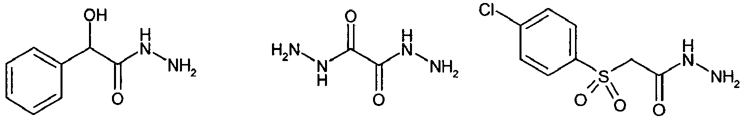

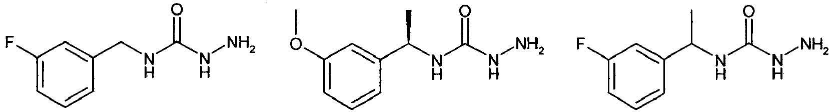

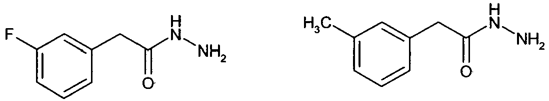

Verbindungen der Formel I gemäß Anspruch 1 können vorzugsweise erhalten werden, indem man Verbindungen der Formel I gemäß Anspruch 1 aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I gemäß Anspruch 1 entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I gemäß Anspruch 1 entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I gemäß Anspruch 1 aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Die Spaltung eines Ethers erfolgt unter Methoden, wie sie dem Fachmann bekannt sind.

Eine Standardmethode zur Etherspaltung, z.B. eines Methylethers, ist die Verwendung von Bortribromid.

Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I gemäß Anspruch 1 werden größtenteils konventionell hergestellt. Sofern die Verbindung der Formel I gemäß Anspruch 1 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I gemäß Anspruch 1, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I gemäß Anspruch 1 mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I gemäß Anspruch 1 können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Salze, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Krebs, Tumorzellen und Tumormetastasen hemmen und sind deshalb für die Tumortherapie geeignet.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antünfektiösen Therapie, zur Steigerung der Lernfähigkeit und Aufmerksamkeit, sowie zur Behandlung und Prophylaxe von Zellalterung und Stress.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankungen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Die Hemmung der SGK1 Proteinkinase kann im Filterbindungsverfahren bestimmt werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray lonization) (M+H)⁺ (wenn nichts anderes angegeben)

### HPLC-Methode

A (polar): Hewlett Pachard System der HP 1100 Serie mit den folgenden Merkmalen: Ionenquelle: ES (positive mode), Scan 100-1000 m/z, Fragmentier-Spannung: 60 V, Gas-Temperatur: 300°C, DAD 220 nm Säule: Chromolith SpeedROD RP-18e, 50-4.6
Flußrate 2.4 ml/min
Der verwendete Splitter reduzierte nach dem DAD die Flußrate für das MS auf 0,75 ml/min
Solvent A: Wasser +0.1% TFA
Solvent B: Acetonitiril + 0.08% TFA

### Gradient:

0.0 min 5% B
2.8 min 100% B
3.3 min 100% B

### B:

Säule: Chromolith SpeedROD RP-18e, 50-4.6
Flußrate 2.4 ml/min

Solvent A: Wasser +0.1 % TFA
Solvent B: Acetonitril + 0.1% TFA

### Gradient:

0.0 min 4% B
2.6 min 100% B
3.3 min 100% B

### Abkürzungen:

DCM = Dichlormethan,
EE = Essigsäureethylester
PE = Petrolether
RT = Raumtemperatur
DAPECI = N-(3-Dimethylaminopropyl)-N'-ethylcarbodiimidhydrochlorid
DMF = Dimethylformamid
HOBT = 1-Hydroxybenzotriazol
NCS = N-Chlorsuccinimid
TFA = Trifluoressigsäure

### Beispiel 1

Die Herstellung von 5-[5-(3-Methoxy-benzyl)-[1,3,4]oxadiazol-2-ylamino]-1*H*-indazol ("A1") erfolgt analog nachstehendem Schema (**Methode 1**)

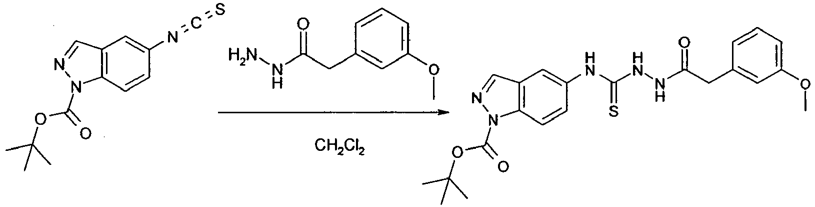

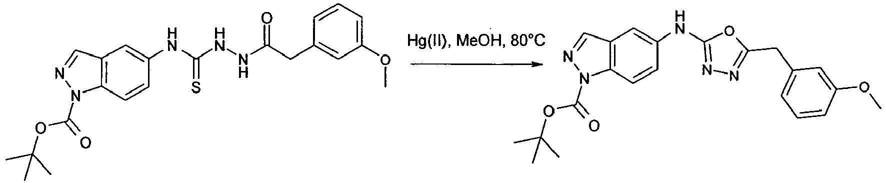

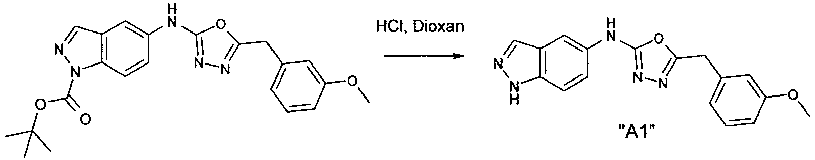

### Schritt 1:

In einem verschlossenden Schraubdeckelgläschen werden 200 mg 5-Isothiocyanato-indazol-1-carbonsäure-tert.-butylester und 140 mg (3-Methoxy-phenyl)-essigsäurehydrazid in 10 ml Dichlormethan bei 50° Außentemperatur 2 Stunden gerührt. Die Reaktionslösung wird eingeengt und das erhaltene Kupplungsprodukt ohne weitere Reinigung für den nächsten Schritt eingesetzt. Man erhält 330 mg Kupplungsprodukt (quant); MS-FAB (M+H⁺) = 456,7; R_{f} (Methode polar): 1,99 Min.

### Schritt 2: Cyclisierung

In einem Schraubdeckelgläschen wird eine Mischung von 319 mg des Kupplungsprodukts aus Schritt 1 mit 286 mg Quecksilber(II)acetat in 10 ml Methanol 1 Stunde bei 80° gerührt. Nach dem Abkühlen wird die Reaktionsmischung über Kieselgel filtriert, mit Methanol nachgewaschen und das erhaltene Filtrat eingeengt. Nach Reinigung durch Säulenchromatographie (Heptan/EE) erhält man 250 mg 5-[5-(3-Methoxy-benzyl)-[1,3,4]oxadiazol-2-ylamino]-indazol-1-carbonsäure-tert.-butylester (85%); MS-FAB (M+H⁺) = 422,7; R_{f} (Methode polar): 2,21 Min.

### Schritt 3: Boc-Abspaltung

In einem Schraubdeckelgläschen werden 250 mg 5-[5-(3-Methoxy-benzyl)-[1,3,4]oxadiazol-2-ylamino]-indazol-1-carbonsäure-tert.-butylester in 10 ml 4N HCl in Dioxan gerührt, bis das Ausgangsmaterial komplett umgesetzt ist. Die Reaktionslösung wird eingeengt und der Rückstand mit Ethylacetat verrüht und abgesaugt. Man erhält 200 mg 5-[5-(3-Methoxy-benzyl)-[1,3,4]oxadiazol-2-ylamino]-1*H*-indazol-Hydrochlorid (94%); MS-FAB (M+H⁺) = 322,34; R_{f} (Methode polar): 1,687 Min.

Analog Methode 1 werden die nachstehenden Verbindungen erhalten

| Nr. | Strukturformel und/oder Name | M+H⁺ | Rf [min] HPLC-Methode polar |
|---|---|---|---|
| "A2" | 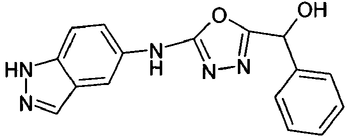 | 308,31 | 1,423 |
| | [5-(1*H*-Indazol-5-ylamino)-[1,3,4]oxadiazol-2-yl]-phenyl-methanol | | |
| "A3" | 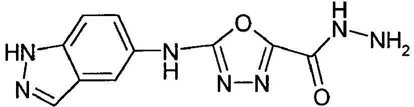 | 260,23 | 1,255 |
| | 5-(1*H*-Indazol-5-ylamino)-[1,3,4]oxadiazol-2-carbonsäure-hydrazid | | |
| "A4" | 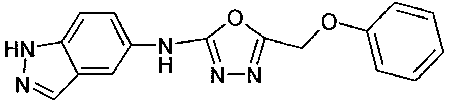 | 308,31 | 1,709 |
| | (1*H*-Indazol-5-yl)-(5-phenoxymethyl-[1,3,4]oxadiazol-2-yl)-amin | | |
| "A5" | 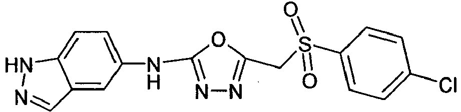 | 390,82 | 1,681 |
| | [5-(4-Chlor-benzolsulfonylmethyl)-[1,3,4]oxadiazol-2-yl]-(1*H*-indazol-5-yl)-amin | | |
| "A6" | 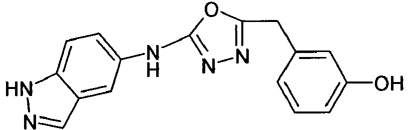 | 308,31 | 1,392 |
| | 3-[5-(1*H*-Indazol-5-ylamino)-[1,3,4]oxadiazol-2-ylmethyl]-phenol | | |
| "A7" | 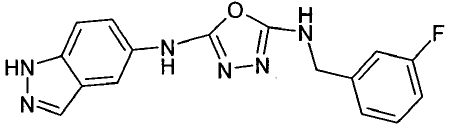 | 325,32 | 1,53 |
| | N-(3-Fluor-benzyl)-N'-(1*H*-indazol-5-yl)-[1,3,4]oxadiazol-2,5-diamin, Hydrochlorid | | |
| "A8" | 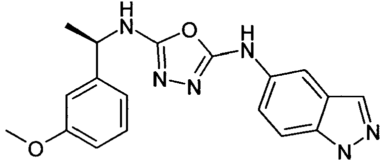 | 351,38 | 1,563 |
| | n N-(1*H*-Indazol-5-yl)-N'-[(R)-1-(3-methoxyphenyl)-ethyl]-[1,3,4]oxadiazol-2,5-diamin | | |
| "A9" | N-(4-Chlor-1*H*-Indazol-5-yl)-N'-[(R)-1-(3-methoxy-phenyl)-ethyl]-[1,3,4]oxadiazol-2,5-diamin, Hydrochlorid | 385,83 | 1,698 |
| "A10" | 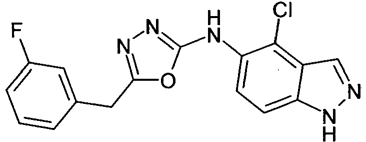 | 344,75 | 1,816 |
| | (4-Chlor-1*H*-indazol-5-yl)-[5-(3-fluor-benzyl)-[1,3,4]oxadiazol-2-yl]-amin, Hydrochlorid | | |
| "A11" | 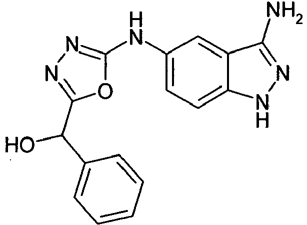 | 323,33 | 2,009 |
| | [5-(3-Amino-1*H*-indazol-5-ylamino)-[1,3,4]oxadiazol-2-yl]-phenyl-methanol, Hydrochlorid | | |
| "A12" | 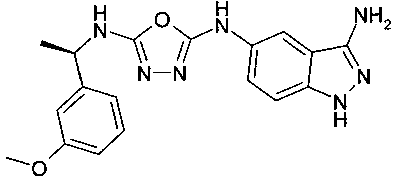 | 366,40 | 2,146 |
| | N5-{5-[(R)-1-(3-Methoxy-phenyl)-ethylamino]-[1,3,4]oxadiazol-2-yl}-1*H*-indazol-3,5-diamin, Hydrochlorid | | |
| "A13" | 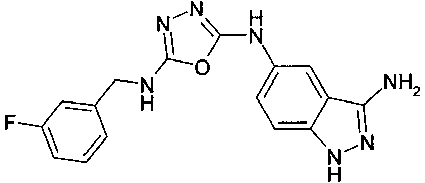 | 340,33 | 2,101 |
| | N5-[5-(3-Fluor-benzylamino)-[1,3,4]oxadiazol-2-yl]-1*H*-indazol-3,5-diamin, Hydrochlorid | | |
| "A14" | 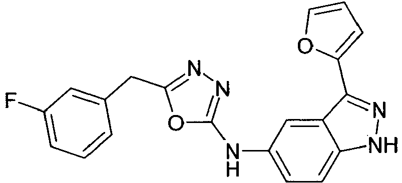 | 376,36 | 2,115 |
| | [5-(3-Fluor-benzyl)-[1,3,4]oxadiazol-2-yl]-(3-furan-2-yl-1*H*-indazol-5-yl)-amin, Hydrochlorid | | |
| "A15" | 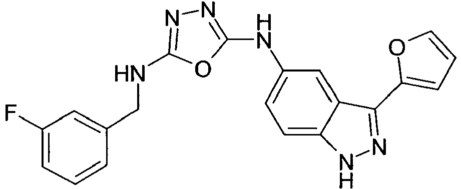 | 391,38 | 2,009 |
| | N-(3-Fluor-benzyl)-N'-(3-furan-2-yl-1*H-*indazol-5-yl)-[1,3,4]oxadiazol-2,5-diamin, Hydrochlorid | | |
| "A16" | 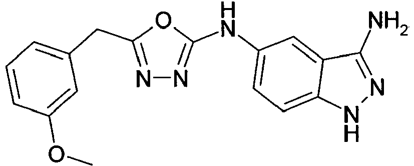 | 337,35 | 1,735 |
| | N5-[5-(3-Methoxy-benzyl)-[1,3,4]oxadiazol-2-yl]-1*H*-ndazol-3,5-diamin | | |
| ¹H-NMR (250 MHz, DMSO-d₆): δ [ppm] 11,26 (1H, s, br), 10,04 (1H, s), 7,87 (1H, s), 7,23-7,32 (2H, m), 7,18 (1H, d, J = 8,8 Hz), 6,82-6,93 (3H, m), 5,17 (2H, s, br), 4,13 (2H, s), 3,75 (3H, s). | | | |
| "A17" | 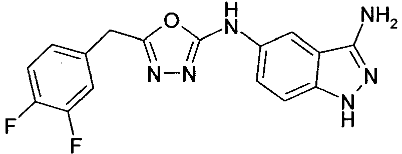 | 343,31 | 1,811 |
| | N5-[5-(3,4-Difluor-benzyl)-[1,3,4]oxadiazol-2-yl]-1*H*-indazol-3,5-diamin | | |
| "A18" | 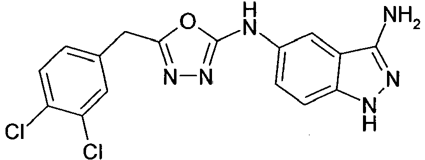 | 376,22 | 1,959 |
| | N5-[5-(3,4-Dichlor-benzyl)-[1,3,4]oxadiazol-2-yl]-1*H*-indazol-3,5-diamin | | |
| "A19" | 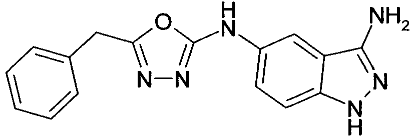 | 307,33 | 1,69 |
| | N5-(5-Benzyl-[1,3,4]oxadiazol-2-yl)-1*H-*indazol-3,5-diamin | | |
| "A20" | 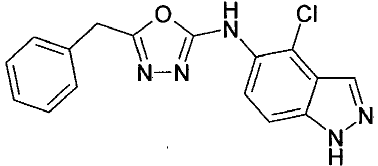 | 326,76 | 2,089 |
| | (5-Benzyl-[1,3,4]oxadiazol-2-yl)-(4-chlor-1 *H-*indazol-5-yl)-amin | | |
| "A21" | 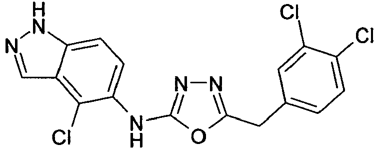 | 395,65 | 2,372 |
| | (4-Chlor-1*H*-indazol-5-yl)-[5-(3,4-dichlor-benzyl)-[1,3,4]oxadiazol-2-yl]-amin | | |
| "A22" | 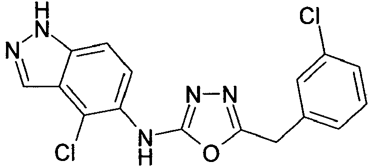 | 361,20 | 2,217 |
| | [5-(3-Chlor-benzyl)-[1,3,4]oxadiazol-2-yl]-(4-chlor-1*H*-indazol-5-yl)-amin | | |
| "A23" | 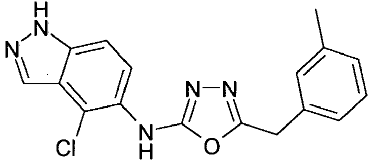 | 340,78 | 2,185 |
| | (4-Chlor-1*H*-indazol-5-yl)-[5-(3-methyl-benzyl)-[1,3,4]oxadiazol-2-yl]-amin | | |
| ¹H-NMR (250 MHz, DMSO-d₆ + TFA-d₁): δ [ppm] 8,13 (1H, s), 7,71 (1H, d, J = 8,9), 7,59 (1H, d, J = 8,9 Hz), 7,27 (1H, t, J = 7,5 Hz), 7,09-7,19 (3H, m), 4,14 (2H, s), 2,32 (3H, s). | | | |
| "A24" | 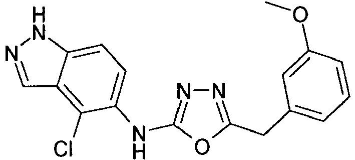 | 356,78 | 2,091 |
| | (4-Chlor-1*H*-indazol-5-yl)-[5-(3-methoxybenzyl)-[1,3,4]oxadiazol-2-yl]-amin | | |
| ¹H-NMR (250 MHz, DMSO-d₆): δ [ppm] 13,38 (1 H, s, br), 9,69 (1H, s), 8,08 (1H, s), 7,69 (1H, d, J = 8,9), 7,53 (1H, d, J = 8,9 Hz), 7,27 (1H, t, J = 7,3 Hz), 6,81-6,92 (3H, m), 4,11 (2H, s), 3,74 (3H, s). | | | |
| "A25" | 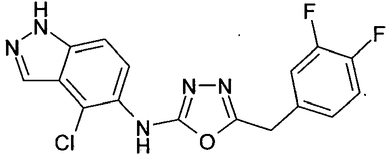 | 362,74 | 2,144 |
| | (4-Chlor-1*H*-indazol-5-yl)-[5-(3,4-difluor-benzyl)-[1,3,4]oxadiazol-2-yl]-amin | | |
| "A26" | 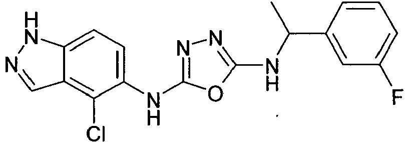 | 373,79 | 1,9 |
| | N-(4-Chlor-1*H*-indazol-5-yl)-N'-[1-(3-fluor-phenyl)-ethyl]-[1,3,4]oxadiazol-2,5-diamin | | |
| "A27" | 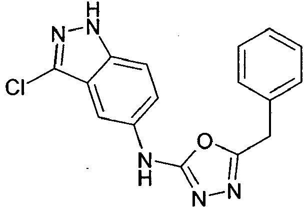 | 326,76 | 1,899 |
| | (5-Benzyl-[1,3,4]oxadiazol-2-yl)-(3-chlor-1*H-*indazol-5-yl)-amin | | |
| "A28" | 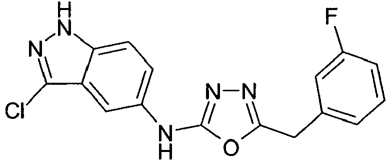 | 344,75 | 1,951 |
| | (3-Chlor-1*H*-indazol-5-yl)-[5-(3-fluor-benzyl)-[1,3,4]oxadiazol-2-yl]-amin | | |
| "A29" | 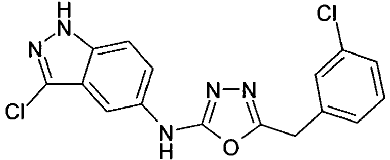 | 361,20 | 2,068 |
| | [5-(3-Chlor-benzyl)-[1,3,4]oxadiazol-2-yl]-(3-chlor-1*H*-indazol-5-yl)-amin | | |
| "A30" | 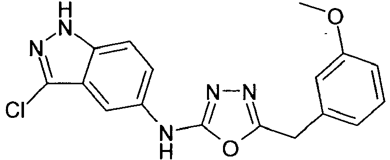 | 356,78 | 1,91 |
| | (3-Chlor-1*H*-indazol-5-yl)-[5-(3-methoxy-benzyl)-[1,3,4]oxadiazol-2-yl]-amin | | |
| "A31" | 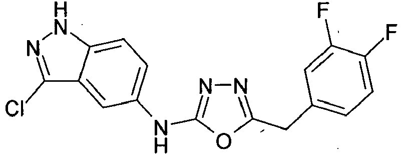 | 362,74 | 1,988 |
| | (3-Chlor-1*H*-indazol-5-yl)-[5-(3,4-difluor-benzyl)-[1,3,4]oxadiazol-2-yl]-amin | | |
| "A32" | 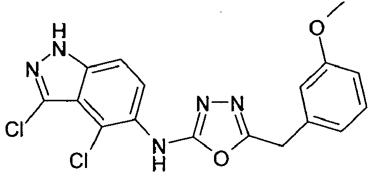 | 391,23 | 2,02 |
| | (3,4-Dichlor-1*H*-indazol-5-yl)-[5-(3-methoxybenzyl)-[1,3,4]oxadiazol-2-yl]-amin | | |
| "A34" | 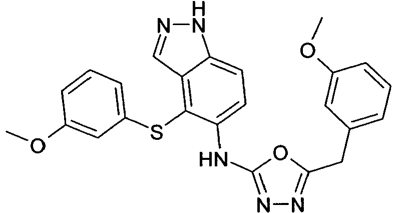 | 460,53 | 2,176 |
| | [5-(3-Methoxy-benzyl)-[1,3,4]oxadiazol-2-yl]-[4-(3-methoxy-phenylsulfanyl)-1*H*-indazol-5-yl]-amin | | |
| "A35" | 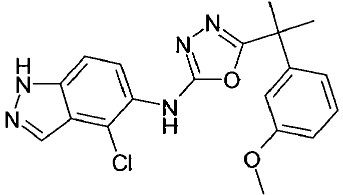 | 384,84 | 1,985 |
| | (4-Chlor-1*H*-indazol-5-yl)-{5-[1-(3-methoxy-phenyl)-1-methyl-ethyl]-[1,3,4]oxadiazol-2-yl}-amin | | |
| "A36" | 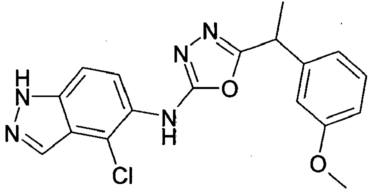 | 370,81 | 1,899 |
| | (4-Chlor-1*H*-indazol-5-yl)-{5-[1-(3-methoxy-phenyl)-ethyl]-[1,3,4]oxadiazol-2-yl}-amin | | |
| "A37" | 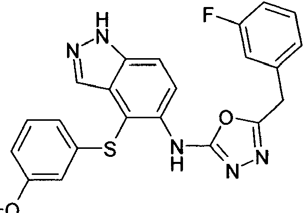 | 448,49 | 2,202 |
| | [5-(3-Fluor-benzyl)-[1,3,4]oxadiazol-2-yl]-[4-(3-methoxy-phenylsulfanyl)-1*H*-indazol-5-yl]-amin | | |
| "A38" | 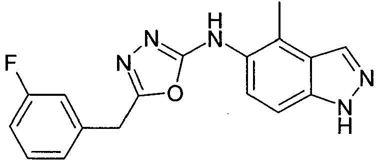 | 360,79 | 1,698 |
| | [5-(3-Fluor-benzyl)-[1,3,4]oxadiazol-2-yl]-(4-methyl-1*H*-indazol-5-yl)-amin, Hydrochlorid | | |
| "A39" | 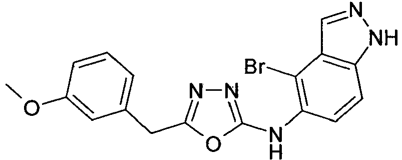 | 401,23 | 1,843 |
| | (4-Brom-1*H*-indazol-5-yl)-[5-(3-methoxy-benzyl)-[1,3,4]oxadiazol-2-yl]-amin, Hydrochlorid | | |
| "A40" | 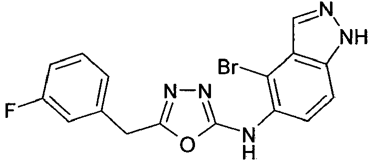 | 389,20 | 1,87 |
| | (4-Brom-1*H*-indazol-5-yl)-[5-(3-fluor-benzyl)-[1,3,4]oxadiazol-2-yl]-amin, Hydrochlorid | | |

### Beispiel 2

Die Herstellung von N3-(1*H*-Indazol-5-yl)-5-(3-methoxy-benzyl)-[1,2,4]triazol-3,4-diamin ("A42") erfolgt analog nachstehendem Schema (**Methode 2**)

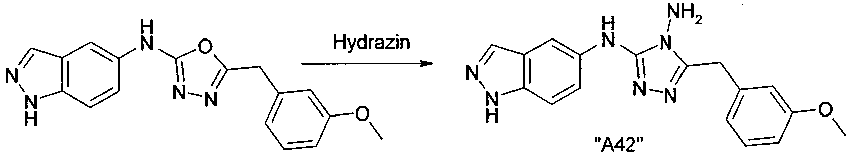

Eine Mischung aus 250 mg 5-[5-(3-Methoxy-benzyl)-[1,3,4]oxadiazol-2-ylamino]-indazol-1-carbonsäure-tert.-butylester (hergestellt nach Methode 1) und 90 mg Hydrazin in 4 ml 1-Butanol werden bei 150° 30 Min. in der Mikrowelle umgesetzt. Die Reaktionsmischung wird eingeengt und durch Säulenchromatographie gereinigt. Man erhält 25 mg "A42" (13%); MS-FAB (M+H⁺) = 336,37; R_{f} (Methode polar): 1,211 Min.

Analog erhält man die Verbindung 5-(4-Chlor-benzolsulfonylmethyl)-N3-(1H-indazol-5-yl)-[1,2,4]triazol-3,4-diamin ("A41"); MS-FAB (M+H⁺) = 404,85; R_{f} (Methode polar): 1,45 Min.

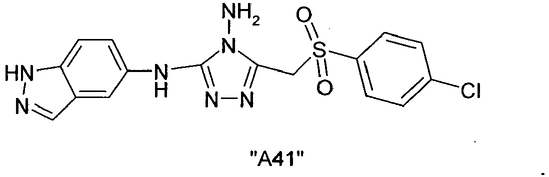

### Beispiel 3

Die Herstellung von [5-(3-Methoxy-benzyl)-[1,3,4]oxadiazol-2-yl]-(3-vinyl-1 H-indazol-5-yl)-amin ("A43") erfolgt analog nachstehendem Schema (**Methode 3**)

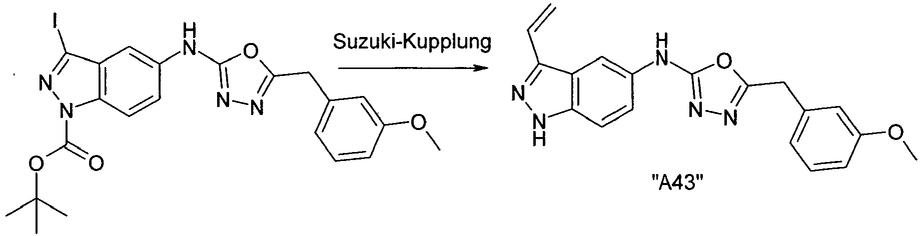

Ein Mischung aus 800 mg 3-lodo-5-[5-(3-methoxy-benzyl)-[1,3,4]oxadiazol-2-ylamino]-indazol-1-carbonsäure-tert.-butylester ("A33", hergestellt nach Methode 1), 173 mg Vinylboronsäuredibutylester, 386 mg Bariumhydroxid (Monohydrat), 173 mg Tetrakis(triphenyl-phosphin)-palladium(0), 40 ml Ethylenglycoldimethylether und 10 ml Wasser wird mehrfach entgast und mit Stickstoff inertisiert. Die Reaktionsmischung wird unter Stickstoff bei 100° Badtemperatur 24 Stunden intensiv gerührt, nach dem Abkühlen auf Wasser gegossen und dreimal mit Essigester extrahiert. Die organische Phase wird mit Wasser gewaschen, getrocknet und zum Rückstand eingeengt. Bei der Reaktion wird die Schutzgruppe bereits abgespalten. Nach Reinigung durch Säulenchromatographie an Kieselgel (Eluent: Heptan/EE) erhält man "A43" (51%); MS-FAB (M+H⁺) = 348,38; R_{f} (Methode polar): 1,854 Min.

Analog erhält man die Verbindung [5-(3-Methoxy-benzyl)-[1,3,4]oxadiazol-2-yl]-[3-((E)-styryl)-1H-indazol-5-yl]-amin ("A44"); MS-FAB (M+H⁺) = 424,47; R_{f} (Methode polar): 2,227 Min.

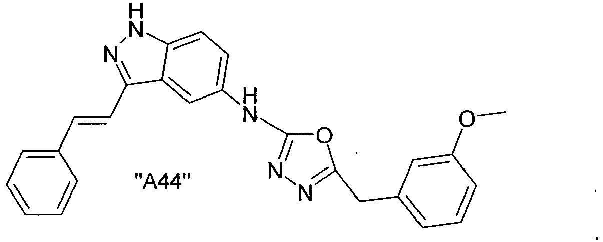

### Beispiel 4

Die Herstellung von (3-Ethyl-1*H*-indazol-5-yl)-[5-(3-methoxy-benzyl)-[1,3,4]oxadiazol-2-yl]-amin ("A45") erfolgt analog nachstehendem Schema (**Methode 4**)

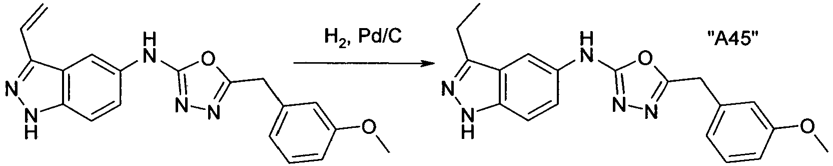

150 mg [5-(3-Methoxy-benzyl)-[1,3,4]oxadiazol-2-yl]-(3-vinyl-1H-indazol-5-yl)-amin (als Trifluoressigsäuresalz, hergestellt aus "A43") werden in 10 ml Methanol in Gegenwart von 150 mg Pd/C bei Raumtemperatur hydriert. Die Reaktionsmischung wird abfiltriert und eingeengt. Nach Reinigung durch Säulenchromatographie an Kieselgel (Eluent: Heptan/EE) erhält man 50 mg (3-Ethyl-1*H*-indazol-5-yl)-[5-(3-methoxy-benzyl)-[1,3,4]oxadiazol-2-yl]-amin (44%); MS-FAB (M+H⁺) = 350,39; R_{f} (Methode polar): 1,836 Min.;
¹H-NMR (250 MHz, DMSO-d₆): δ [ppm]12,49 (1H, s, br), 10,21 (1H, s), 7,99 (1H, s), 7,41 (1H, d, J = 8,8), 7,32 (1H, d, J = 8,8 Hz), 7,28 (1H, t, J = 7,8 Hz), 6,88-6,92 (3H, m), 4,14 (2H, s), 3,75 (3H, s), 2,88 (2H, q, J = 7,7 Hz), 1,31 (3H, t, J = 7,7 Hz).

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO4 · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatine kapseln gefüllt, so daß jede Kapsel 20 mg des Wirkstoffs enthält.

### Beispiel H: Ampullen

Eine Lösung von 1 kg eines erfindungsgemäßen Wirkstoffes in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Nr. | Strukturformel und/oder Name |
|---|---|
| "A1" | 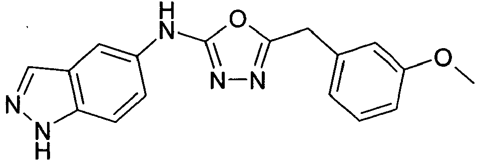 |
| | 5-[5-(3-Methoxy-benzyl)-[1,3,4]oxadiazol-2-ylamino]-1*H-*indazol |
| "A2" | 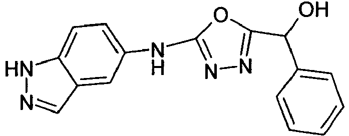 |
| | [5-(1*H*-Indazol-5-ylamino)-[1,3,4]oxadiazol-2-yl]-phenyl-methanol |
| "A3" | 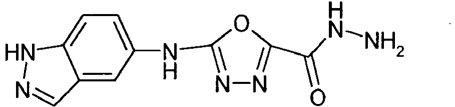 |
| | 5-(1*H*-Indazol-5-ylamino)-[1,3,4]oxadiazol-2-carbonsäurehydrazid |
| "A4" | 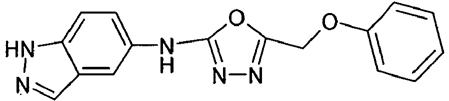 |
| | (1*H*-Indazol-5-yl)-(5-phenoxymethyl-[1,3,4]oxadiazol-2-yl)-amin |
| "A5" | 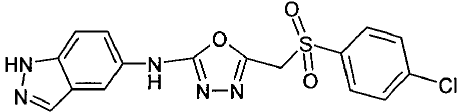 |
| | [5-(4-Chlor-benzolsulfonylmethyl)-[1,3,4]oxadiazol-2-yl]-(1*H-*indazol-5-yl)-amin |
| "A6" | 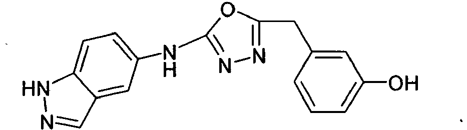 |
| | 3-[5-(1*H*-Indazol-5-ylamino)-[1,3,4]oxadiazol-2-yfmethyl]-phenol |
| "A7" | 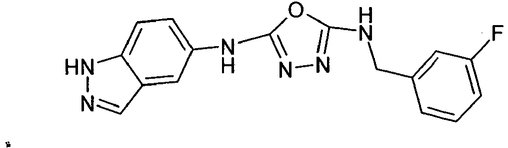 |
| | N-(3-Fluor-benzyl)-N'-(1*H*-indazol-5-yl)-[1,3,4]oxadiazol-2,5-diamin, Hydrochlorid |
| "A8" | 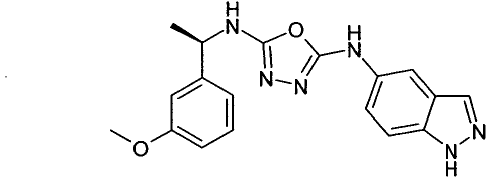 |
| | N-(1*H*-Indazol-5-yl)-N'-[(R)-1-(3-methoxy-phenyl)-ethyl]-[1,3,4]oxadiazol-2,5-diamin |
| "A9" | N-(4-Chlor-1*H*-Indazol-5-yl)-N'-[(R)-1-(3-methoxy-phenyl)-ethyl]-[1,3,4]oxadiazol-2,5-diamin |
| "A10" | 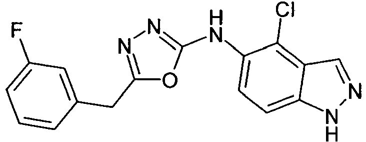 |
| | (4-Chlor-1*H*-indazol-5-yl)-[5-(3-fluor-benzyl)-[1,3,4]oxadiazol-2-yl]-amin |
| "A11" | 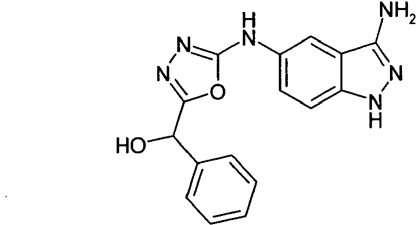 |
| | [5-(3-Amino-1*H*-indazol-5-ylamino)-[1,3,4]oxadiazol-2-yl]-phenyl-methanol |
| "A12" | 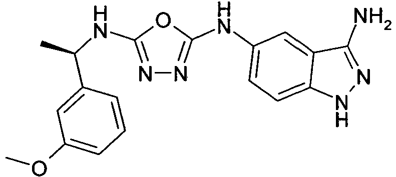 |
| | N5-{5-[(R)-1-(3-Methoxy-phenyl)-ethylamino]-[1,3,4]oxadiazol-2-yl}-1*H*-indazol-3,5-diamin |
| "A13" | 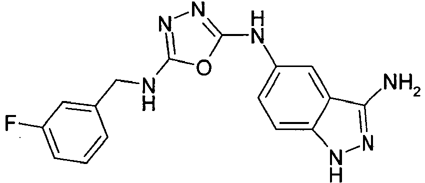 |
| | N5-[5-(3-Fluor-benzylamino)-[1,3,4]oxadiazol-2-yl]-1*H-*indazol-3,5-diamin |
| "A14" | 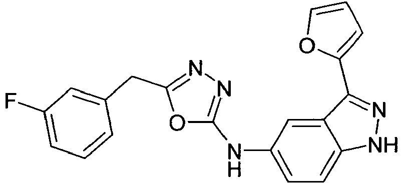 |
| | [5-(3-Fluor-benzyl)-[1,3,4]oxadiazol-2-yl]-(3-furan-2-yl-1*H-*indazol-5-yl)-amin |
| "A15" | 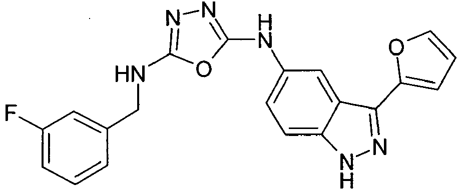 |
| | N-(3-Fluor-benzyl)-N'-(3-furan-2-yl-1*H*-indazol-5-yl)-[1,3,4]oxadiazol-2,5-diamin |
| "A16" | 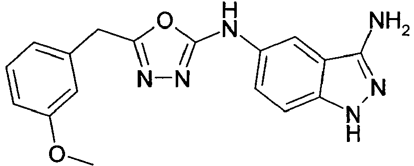 |
| | N5-[5-(3-Methoxy-benzyl)-[1,3,4]oxadiazol-2-yl]-1*H*-indazol-3,5-diamin |
| "A17" | 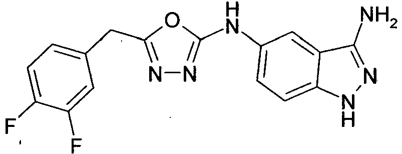 |
| | N5-[5-(3,4-Difluor-benzyl)-[1,3,4]oxadiazol-2-yl]-1*H*-indazol-3,5-diamin |
| "A18" | 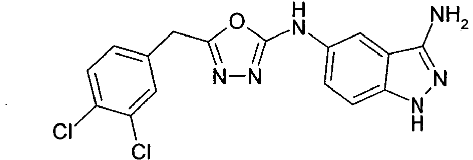 |
| | N5-[5-(3,4-Dichlor-benzyl)-[1,3,4]oxadiazol-2-yl]-1*H*-indazol-3,5-diamin |
| "A19" | 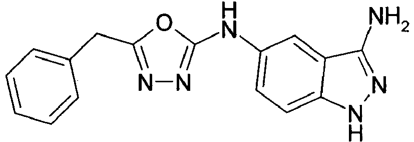 |
| | N5-(5-Benzyl-[1,3,4]oxadiazol-2-yl)-1*H*-indazol-3,5-diamin |
| "A20" | 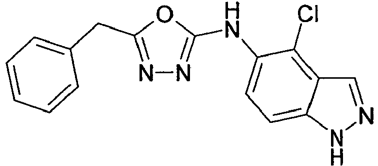 |
| | (5-Benzyl-[1,3,4]oxadiazol-2-yl)-(4-chlor-1*H*-indazol-5-yl)-amin |
| "A21" | 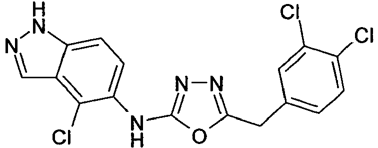 |
| | (4-Chlor-1*H*-indazol-5-yl)-[5-(3,4-dichlor-benzyl)-[1,3,4]oxadiazol-2-yl]-amin |
| "A22" | 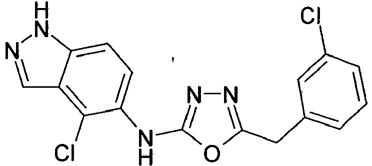 |
| | [5-(3-Chlor-benzyl)-[1',3,4]oxadiazol-2-yl]-(4-chlor-1*H-*indazol-5-yl)-amin |
| "A23" | 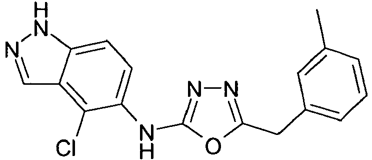 |
| | (4-Chlor-1*H*-indazol-5-yl)-[5-(3-methyl-benzyl)-[1,3,4]oxadiazol-2-yl]-amin |
| "A24" | 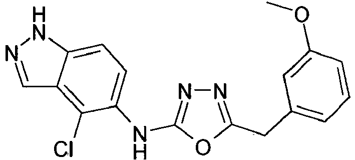 |
| | (4-Chlor-1*H*-indazol-5-yl)-[5-(3-methoxy-benzyl)-[1,3,4]oxadiazol-2-yl]-amin |
| "A25" | 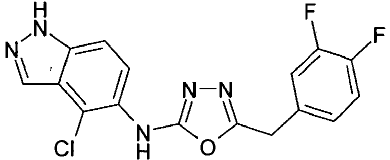 |
| | (4-Chlor-1*H*-indazol-5-yl)-[5-(3,4-difluor-benzyl)-[1,3,4]oxadiazol-2-yl]-amin |
| "A26" | 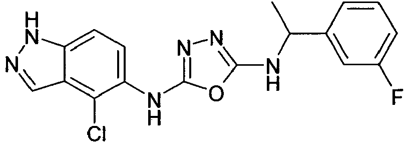 |
| | N-(4-Chlor-1*H*-indazol-5-yl)-N'-[1-(3-fluor-phenyl)-ethyl]-[1,3,4]oxadiazol-2,5-diamin |
| "A27" | 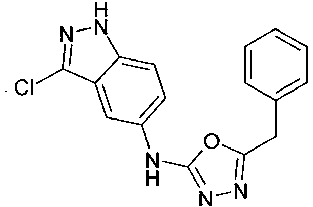 |
| | (5-Benzyl-[1,3,4]oxadiazol-2-yl)-(3-chlor-1*H*-indazol-5-yl)-amin |
| "A28" | 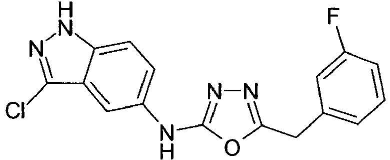 |
| | (3-Chlor-1*H*-indazol-5-yl)-[5-(3-fluor-benzyl)-[1,3,4]oxadiazol-2-yl]-amin |
| "A29" | 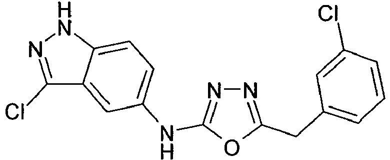 |
| | [5-(3-Chlor-benzyl)-[1,3,4]oxadiazol-2-yl]-(3-chlor-1*H-*indazol-5-yl)-amin |
| "A30" | 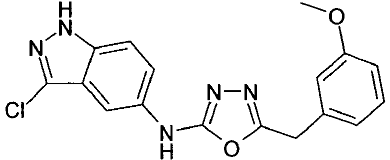 |
| | (3-Chlor-1*H*-indazol-5-yl)-[5-(3-methoxy-benzyl)-[1,3,4]oxadiazol-2-yl]-amin |
| "A31" | 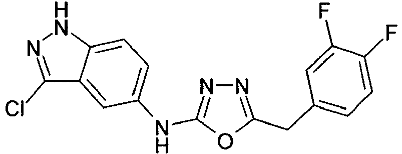 |
| | (3-Chlor-1*H*-indazol-5-yl)-[5-(3,4-difluor-benzyl)-[1,3,4]oxadiazol-2-yl]-amin |
| "A32" | 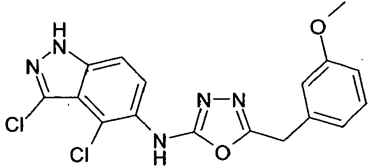 |
| | (3,4-Dichlor-1*H*-indazol-5-yl)-[5-(3-methoxy-benzyl)-[1,3,4]oxadiazol-2-yl]-amin |
| "A33" | 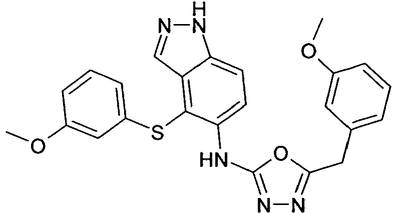 |
| | [5-(3-Methoxy-benzyl)-[1,3,4]oxadiazol-2-yl]-[4-(3-methoxy-phenylsulfanyl)-1*H*-indazol-5-yl]-amin |
| "A34" | 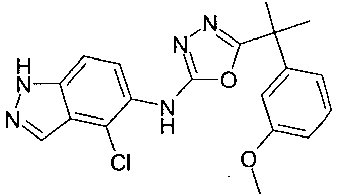 |
| | (4-Chlor-1*H*-indazol-5-yl)-{5-[1-(3-methoxy-phenyl)-1-methyl-ethyl]-[1,3,4]oxadiazol-2-yl}-amin |
| "A35" | 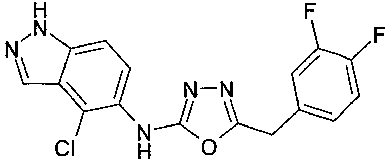 |
| | (4-Chlor-1*H*-indazol-5-yl)-[5-(3,4-difluor-benzyl)-[1,3,4]oxadiazol-2-yl]-amin |
| "A36" | 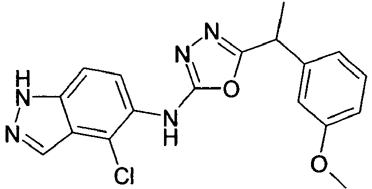 |
| | (4-Chlor-1*H*-indazol-5-yl)-{5-[1-(3-methoxy-phenyl)-ethyl]-[1,3,4]oxadiazol-2-yl}-amin |
| "A37" | 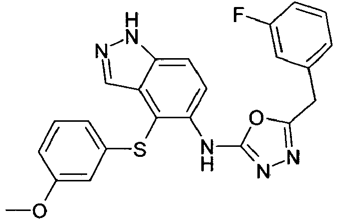 |
| | [5-(3-Fluor-benzyl)-[1,3,4]oxadiazol-2-yl]-[4-(3-methoxy-phenylsulfanyl)-1*H*-indazol-5-yl]-amin |
| "A38" | 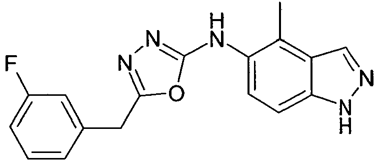 |
| | [5-(3-Fluor-benzyl)-[1,3,4]oxadiazol-2-yl]-(4-methyl-1*H-*indazol-5-yl)-amin |
| "A39" | 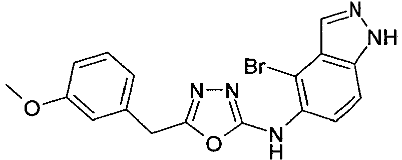 |
| | (4-Brom-1*H*-indazol-5-yl)-[5-(3-methoxy-benzyl)-[1,3,4]oxadiazol-2-yl]-amin |
| "A40" | 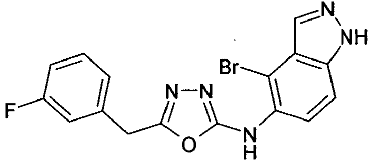 |
| | (4-Brom-1*H*-indazol-5-yl)-[5-(3-fluor-benzyl)-[1,3,4]oxadiazol-2-yl]-amin |
| "A41" | 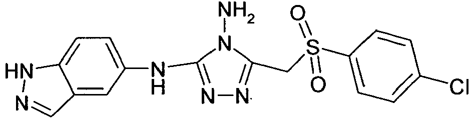 |
| | 5-(4-Chlor-benzolsulfonylmethyl)-N3-(1*H*-indazol-5-yl)-[1,2,4]triazol-3,4-diamin |
| "A42" | 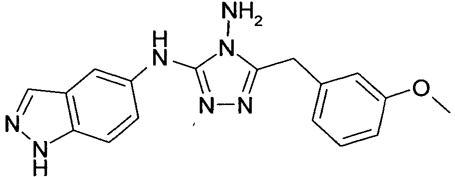 |
| | N3-(1*H*-Indazof-5-yl)-5-(3-methoxy-benzyl)-[1,2,4]triazol-3,4-diamin |
| "A43" | 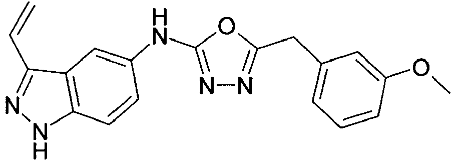 |
| | [5-(3-Methoxy-benzyl)-[1,3,4]oxadiazol-2-yl]-(3-vinyl-1*H-*indazol-5-yl)-amin |
| "A44" | 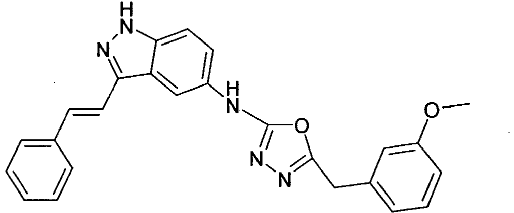 |
| | [5-(3-Methoxy-benzyl)-[1,3,4]oxadiazol-2-yl]-[3-((E)-styryl)-1H-indazol-5-yl]-amin |
| "A45" | 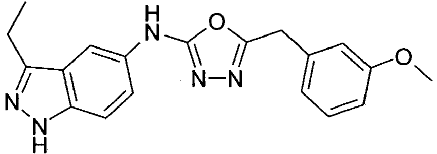 |
| | (3-Ethyl-1*H*-indazol-5-yl)-[5-(3-methoxy-benzyl)-[1,3,4]oxadiazol-2-yl]-amin |
sowie ihre pharmazeutisch verwendbaren Salze, Solvate und Stereoisomere, einschließlich deren Mischungen in allen Verhältnissen.

2. Arzneimittel, enthaltend mindestens eine Verbindung nach Anspruch 1-und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

3. Verwendung von Verbindungen gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress und zur Behandlung von Tinitus.

4. Verwendung nach Anspruch 3, wobei es sich bei Diabetes um Diabetes mellitus, diabetische Nephropathie, diabetische Neuropathie, diabetische Angiopathie und Mikroangiopathie handelt.

5. Verwendung nach Anspruch 3, wobei es sich bei Herzkreislauferkrankungen um kardiale Fibrosen nach Myokardinfarkt, Herzhypertrophie, Herzinsuffizienz und Arteriosklerose handelt.

6. Verwendung nach Anspruch 3, wobei es sich bei Nierenerkrankungen um Glomerulosklerose, Nephrosklerose, Nephritis, Nephropathie und Störung der Elektrolytausscheidung handelt.

7. Verwendung nach Anspruch 3, wobei es sich bei Fibrosen und entzündlichen Prozessen um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung und Morbus Alzheimer handelt.

8. Arzneimittel enthaltend mindestens eine Verbindung gemäß Anspruch 1 und/oder ihre pharmazeutisch verwendbaren Salze, Solvate und Stereoisomere, einschließlich deren Mischungen in allen Verhältnissen, und mindestens einen weiteren Arzneimittelwirkstoff.

9. Set (Kit), bestehend aus getrennten Packungen von
(a) einer wirksamen Menge an einer Verbindung gemäß Anspruch 1 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

## Claims

1. Compounds selected from the group
| No. | Structural formula and/or name |
|---|---|
| "A1" | 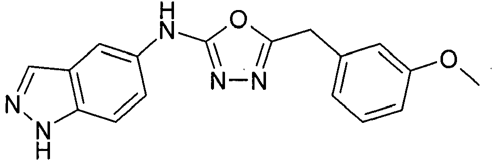 |
| | 5-[5-(3-Methoxybenzyl)-[1,3,4]oxadiazol-2-ylamino]-1 *H-*indazole |
| "A2" | 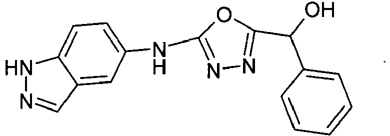 |
| | [5-(1*H*-Indazol-5-ylamino)-[1,3,4]oxadiazol-2-yl]phenyl-methanol |
| "A3" | 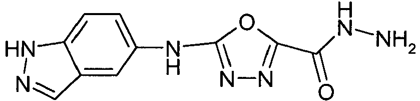 |
| | 5-(1*H*-Indazol-5-ylamino)-[1,3,4]oxadiazole-2-carbohydrazide |
| "A4" | 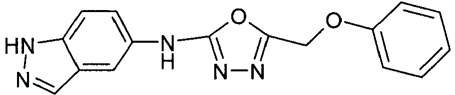 |
| | (1*H*-Indazol-5-yl)-(5-phenoxymethyl-[1,3,4]oxadiazol-2-yl)-amine |
| "A5" | 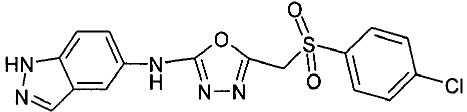 |
| | [5-(4-Chlorobenzenesulfonylmethyl)-[1,3,4]oxadiazol-2-yl]-(1*H*-indazol-5-yl)amine |
| "A6" | 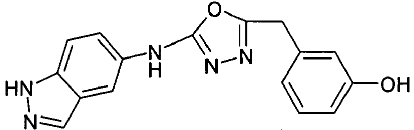 |
| | 3-[5-(1*H*-Indazol-5-ylamino)-[1,3,4]oxadiazol-2-ylmethyl]-phenol |
| "A7" | 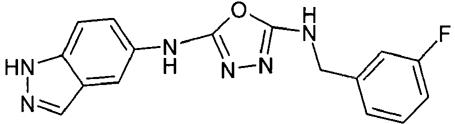 |
| | N(3-Fluorobenzyl)-N'-(1*H*-indazol-5-yl)-[1,3,4]oxadiazole-2,5-diamine, hydrochloride |
| "A8" | 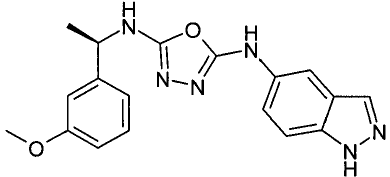 |
| | N-(1*H*-Indazol-5-yl)-N'-[(R)-1-(3-methoxyphenyl)ethyl]-[1,3,4]oxadiazole-2,5-diamine |
| "A9" | N-(4-Chloro-1*H*-indazol-5-yl)-N'-[(R)-1-(3-methoxyphenyl)-ethyl]-[1,3,4]oxadiazole-2,5-diamine |
| "A10" | 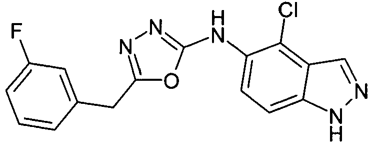 |
| | (4-Chloro-1*H*-indazol-5-yl)-[5-(3-fluorobenzyl)-[1,3,4]oxadiazol-2-yl]amine |
| "A11" | 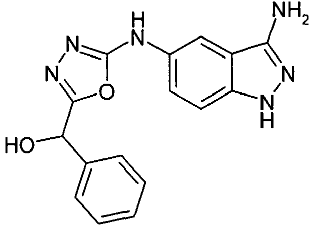 |
| | [5-(3-Amino-1*H*-indazol-5-ylamino)-[1,3,4]oxadiazol-2-yl]-phenylmethanol |
| "A12" | 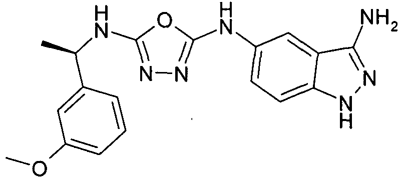 |
| | N5-{5-[(R)-1-(3-Methoxyphenyl)ethylamino]-[1,3,4]oxadiazol-2-yl}-1*H*-indazole-3,5-diamine |
| "A13" | 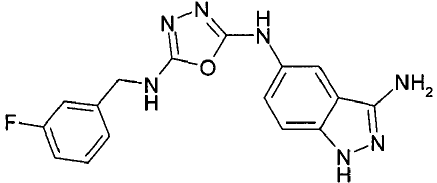 |
| | N5-[5-(3-Fluorobenzylamino)-[1,3,4]oxadiazol-2-yl]-1*H-*indazole-3,5-diamine |
| "A14" | 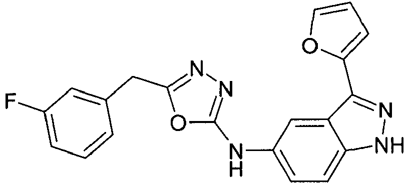 |
| | [5-(3-Fluorobenzyl)-[1,3,4]oxadiazol-2-yl]-(3-furan-2-yl-1*H-*indazol-5-yl)amine |
| "A15" | 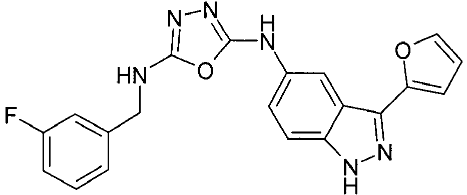 |
| | N-(3-Fluorobenzyl)-N'-(3-furan-2-yl-1*H*-indazol-5-yl)-[1,3,4]oxadiazole-2,5-diamine |
| "A16" | 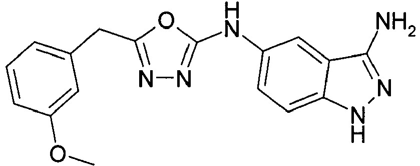 |
| | N5-[5-(3-Methoxybenzyl)-[1,3,4]oxadiazol-2-yl]-1*H*-indazole-3,5-diamine |
| "A17" | 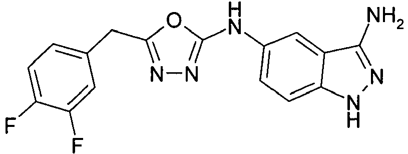 |
| | N5-[5-(3,4-Difluorobenzyl)-[1,3,4]oxadiazol-2-yl]-1 *H-*indazole-3,5-diamine |
| "A18" | 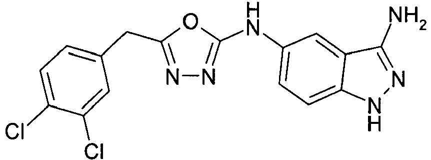 |
| | N5-[5-(3,4-Dichlorobenzyl)-[1,3,4]oxadiazol-2-yl]-1 *H-*indazole-3,5-diamine |
| "A19" | 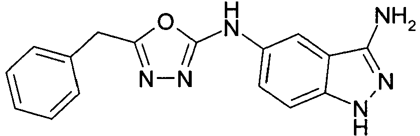 |
| | N5-(5-Benzyl-[1,3,4]oxadiazol-2-yl)-1*H*-indazole-3,5-diamine |
| "A20" | 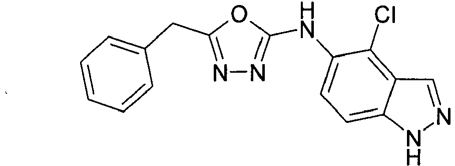 |
| | (5-Benzyl-[1,3,4]oxadiazol-2-yl)-(4-chloro-1*H*-indazol-5-yl)-amine |
| "A21" | 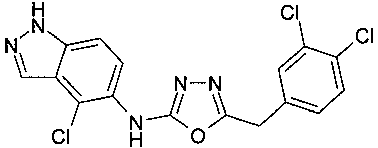 |
| | (4-Chloro-1*H*-indazol-5-yl)-[5-(3,4-dichlorobenzyl)-[1,3,4]oxadiazol-2-yl]amine |
| "A22" | 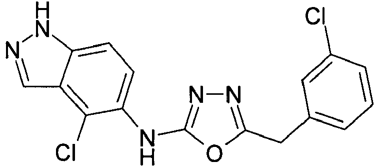 |
| | [5-(3-Chlorobenzyl)-[1,3,4]oxadiazol-2-yl]-(4-chloro-1*H-*indazol-5-yl)amine |
| "A23" | 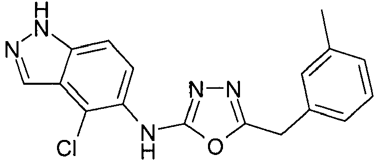 |
| | (4-Chloro-1*H*-indazol-5-yl)-[5-(3-methylbenzyl)-[1,3,4]oxadiazol-2-yl]amine |
| "A24" | 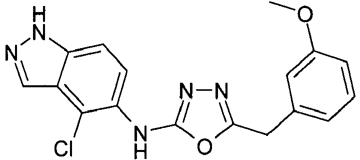 |
| | (4-Chloro-1*H*-indazol-5-yl)-[5-(3-methoxybenzyl)-[1,3,4]oxadiazol-2-yl]amine |
| "A25" | 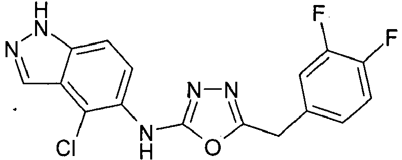 |
| | (4-Chloro-1*H*-indazol-5-yl)-[5-(3,4-difluorobenzyl)-[1,3,4]oxadiazol-2-yl]amine |
| "A26" | 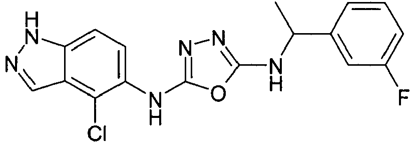 |
| | N-(4-Chloro-1*H*-indazol-5-yl)-N'-[1-(3-fluorophenyl)ethyl]-[1,3,4]oxadiazole-2,5-diamine |
| "A27" | 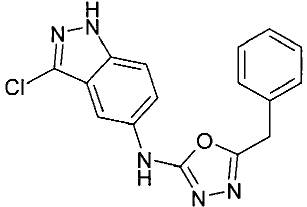 |
| | (5-Benzyl-[1,3,4]oxadiazol-2-yl)-(3-chloro-1*H*-indazol-5-yl)-amine |
| "A28" | 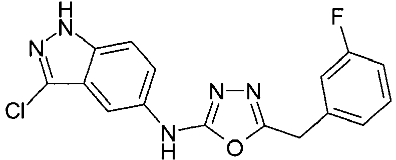 |
| | (3-Chloro-1*H*-indazol-5-yl)-[5-(3-fluorobenzyl)-[1,3,4]oxadiazol-2-yl]amine |
| "A29" | 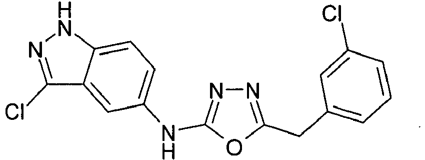 |
| | [5-(3-Chlorobenzyl)-[1,3,4]oxadiazol-2-yl]-(3-chloro-1*H-*indazol-5-yl)amine |
| "A30" | 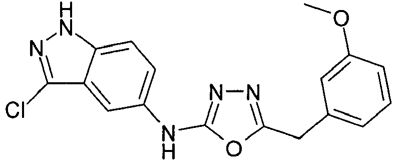 |
| | (3-Chloro-1*H*-indazol-5-yl)-[5-(3-methoxybenzyl)-[1,3,4]oxadiazol-2-yl]amine |
| "A31" | 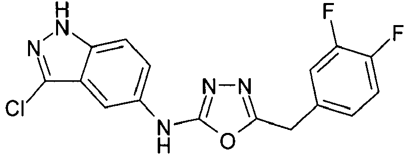 |
| | (3-Chloro-1*H*-indazol-5-yl)-[5-(3,4-difluorobenzyl)-[1,3,4]oxadiazol-2-yl]amine |
| "A32" | 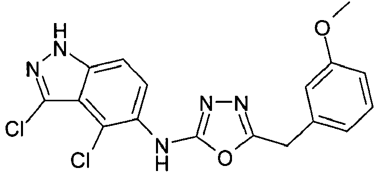 |
| | (3,4-Dichloro-1*H*-indazol-5-yl)-[5-(3-methoxybenzyl)-[1,3,4]oxadiazol-2-yl]amine |
| "A33" | 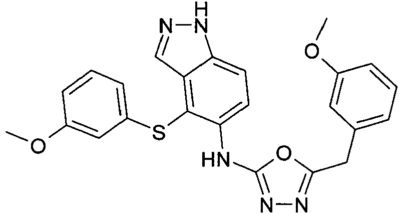 |
| | [5-(3-Methoxybenzyl)-[1,3,4]oxadiazol-2-yl]-[4-(3-methoxy-phenylsulfanyl)-1*H*-indazol-5-yl]amine |
| "A34" | 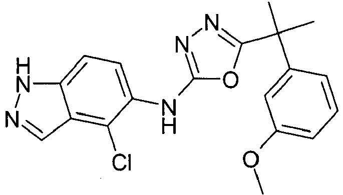 |
| | (4-Chloro-1*H*-indazol-5-yl)-{5-[1-(3-methoxyphenyl)-1-methylethyl]-[1,3,4]oxadiazol-2-yl}amine |
| "A35" | 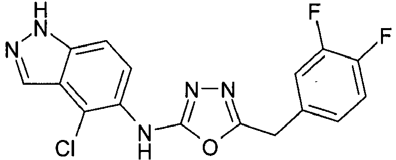 |
| | (4-Chloro-1*H*-indazol-5-yl)-[5-(3,4-difluorobenzyl)-[1,3,4]oxadiazol-2-yl]amine |
| "A36" | 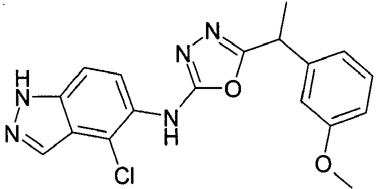 |
| | (4-Chloro-1*H*-indazol-5-yl)-{5-[1-(3-methoxyphenyl)ethyl]-[1,3,4]oxadiazol-2-yl}amine |
| "A37" | 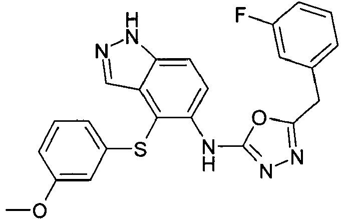 |
| | [5-(3-Fluorobenzyl)-[1,3,4]oxadiazol-2-yl]-[4-(3-methoxy-phenylsulfanyl)-1*H*-indazol-5-yl]amine |
| "A38" | 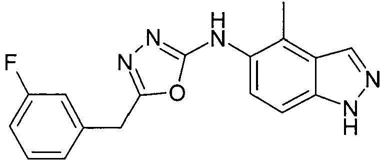 |
| | [5-(3-Fluorobenzyl)-[1,3,4]oxadiazol-2-yl]-(4-methyl-1*H-*indazol-5-yl)amine |
| "A39" | 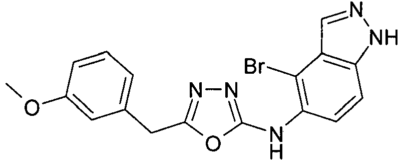 |
| | (4-Bromo-1*H*-indazol-5-yl)-[5-(3-methoxybenzyl)-[1,3,4]oxadiazol-2-yl]amine |
| "A40" | 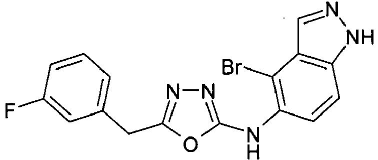 |
| | (4-Bromo-1*H*-indazol-5-yl)-[5-(3-fluorobenzyl)-[1,3,4]oxadiazol-2-yl]amine |
| "A41" | 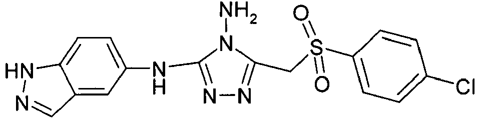 |
| | 5-(4-Chlorobenzenesulfonylmethyl)-N3-(1*H*-indazol-5-yl)-[1,2,4]triazole-3,4-diamine |
| "A42" | 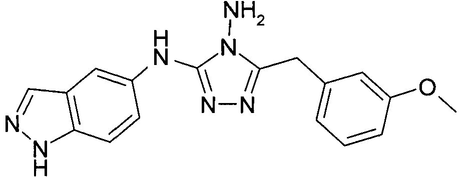 |
| | N3-(1*H*-Indazol-5-yl)-5-(3-methoxybenzyl)-[1,2,4]triazole-3,4-diamine |
| "A43" | 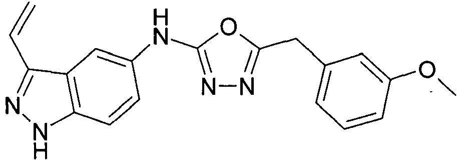 |
| | [5-(3-Methoxybenzyl)-[1,3,4]oxadiazol-2-yl]-(3-vinyl-1H-indazol-5-yl)amine |
| "A44" | 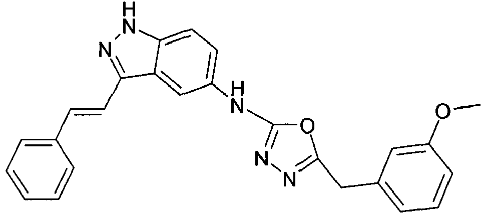 |
| | [5-(3-Methoxybenzyl)-[1,3,4]oxadiazol-2-yl]-[3-((E)-styryl)-1H-indazol-5-yl]amine |
| "A45" | 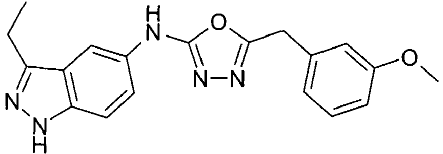 |
| | (3-Ethyl-1*H*-indazol-5-yl)-[5-(3-methoxybenzyl)-[1,3,4]oxadiazol-2-yl]amine |
and pharmaceutically usable salts, solvates and stereoisomers thereof, including mixtures thereof in all ratios.

2. Medicaments comprising at least one compound according to Claim 1 and/or pharmaceutically usable salts, solvates and stereoisomers thereof, including mixtures thereof in all ratios, and optionally excipients and/or adjuvants.

3. Use of compounds according to Claim 1, and pharmaceutically usable salts, solvates and stereoisomers thereof, including mixtures thereof in all ratios, for the preparation of a medicament for the treatment or prevention of diabetes, obesity, metabolic syndrome (dyslipidaemia), systemic and pulmonary hypertonia, cardiovascular diseases and kidney diseases, generally in fibroses and inflammatory processes of any type, cancer, tumour cells, tumour metastases, coagulopathies, neuronal excitability, glaucoma, cataract, bacterial infections and in antiinfection therapy, for increasing learning ability and attention, and for the treatment and prophylaxis of cell ageing and stress and for the treatment of tinnitus.

4. Use according to Claim 3, where diabetes is diabetes mellitus, diabetic nephropathy, diabetic neuropathy, diabetic angiopathy and microangiopathy.

5. Use according to Claim 3, where cardiovascular diseases are cardiac fibroses after myocardial infarction, cardiac hypertrophy, cardiac insufficiency and arteriosclerosis.

6. Use according to Claim 3, where kidney diseases are glomerulosclerosis, nephrosclerosis, nephritis, nephropathy and electrolyte excretion disorder.

7. Use according to Claim 3, where fibroses and inflammatory processes are liver cirrhosis, pulmonary fibrosis, fibrosing pancreatitis, rheumatism and arthroses, Crohn's disease, chronic bronchitis, radiation fibrosis, sclerodermatitis, cystic fibrosis, scarring and Alzheimer's disease.

8. Medicaments comprising at least one compound according to Claim 1 and/or pharmaceutically usable salts, solvates and stereoisomers thereof, including mixtures thereof in all ratios, and at least one further medicament active compound.

9. Set (kit) consisting of separate packs of
(a) an effective amount of a compound according to Claim 1 and/or pharmaceutically usable salts, solvates and stereoisomers thereof, including mixtures thereof in all ratios,
and
(b) an effective amount of a further medicament active compound.

## Revendications

1. Composés choisis dans le groupe constitué par
| n° | Formule structurelle et/ou nom |
|---|---|
| « A1 » | 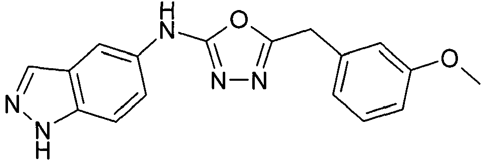 |
| | 5-[5-(3-Méthoxybenzyl)-[1,3,4]oxadiazol-2-ylamino]-1*H-*indazole |
| « A2 » | 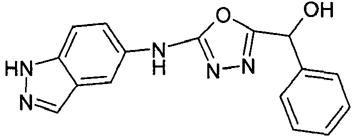 |
| | [5-(1*H*-Indazol-5-ylamino)-[1,3,4]oxadiazol-2-yl]-phénylméthanol |
| « A3 » | 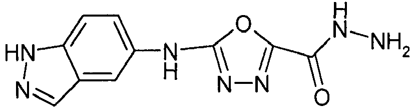 |
| | 5-(1*H*-Indazol-5-ylamino)-[1,3,4]oxadiazole-2-carbohydrazide |
| « A4 » | 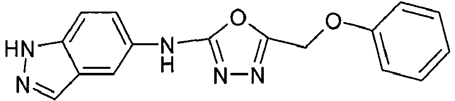 |
| | (1*H*-Indazol-5-yl)-(5-phénoxyméthyl-[1,3,4]oxadiazol-2-yl)amine |
| « A5 » | 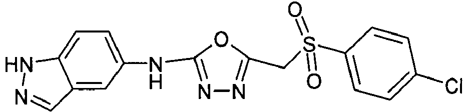 |
| | [5-(4-Chlorobenzènesulfonylméthyl)-[1,3,4]oxadiazol-2-yl]-(1*H*-indazol-5-yl)amine |
| « A6 » | 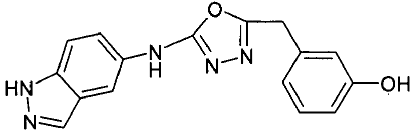 |
| | 3-[5-(1*H*-Indazol-5-ylamino)-[1,3,4]oxadiazol-2-ylméthyl]phénol |
| « A7 » | 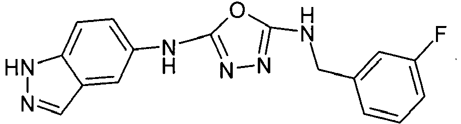 |
| | N-(3-Fluorobenzyl)-N'-(1*H*-indazol-5-yl)-[1,3,4]oxadiazole-2,5-diamine, chlorhydrate |
| « A8 » | 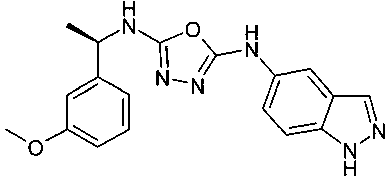 |
| | N-(1*H*-Indazol-5-yl)-N'-[(R)-1-(3-méthoxyphényl)éthyl]-[1,3,4]oxadiazole-2,5-diamine |
| « A9 » | N-(4-Chloro-1*H*-indazol-5-yl)-N'-[(R)-1-(3-méthoxyphényl)-éthyl]-[1,3,4]oxadiazole-2,5-diamine |
| « A10 » | 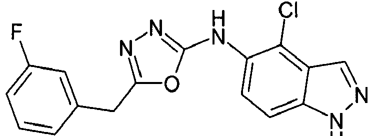 |
| | (4-Chloro-1*H*-indazol-5-yl)-[5-(3-fluorobenzyl)- [1,3,4]oxadiazol-2-yl]amine-[1,3,4]oxadiazol-2-yl]amine |
| « A11 » | 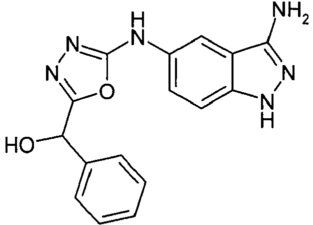 |
| | [5-(3-Amino-1*H*-indazol-5-ylamino)-[1,3,4]oxadiazol-2-yl]-phénylméthanol |
| «A12» | 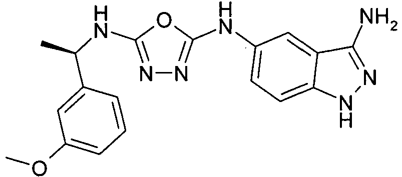 |
| | N5-{5-[(R)-1-(3-Méthoxyphényl)éthylamino]-[1,3,4]oxadiazol-2-yl}-1*H*-indazole-3,5-diamine |
| « A13 » | 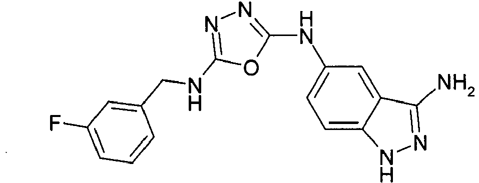 |
| | N5-[5-(3-Fluorobenzylamino)-[1,3,4]oxadiazol-2-yl]-1*H-*indazole-3,5-diamine |
| «A14» | 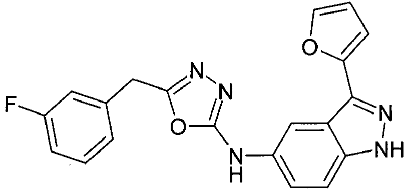 |
| | [5-(3-Fluorobenzyl)-[1,3,4]oxadiazol-2-yl]-(3-furan-2-yl-1*H-*indazol-5-yl)amine |
| « A15 » | 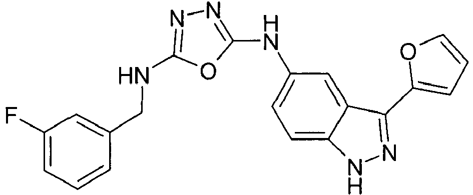 |
| | N-(3-Fluorobenzyl)-N'-(3-furan-2-yl-1*H*-indazol-5-yl)-[1,3,4]oxadiazole-2,5-diamine |
| « A16 » | 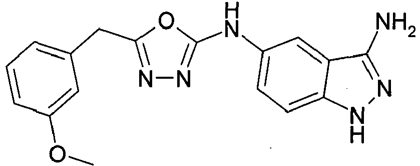 |
| | N5-[5-(3-Méthoxybenzyl)-[1,3,4]oxadiazol-2-yl]-1*H*-indazole-3,5-diamine |
| « A17 » | 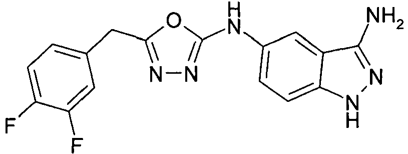 |
| | N5-[5-(3,4-Difluorobenzyl)-[1,3,4]oxadiazol-2-yl]-1*H-*indazole-3,5-diamine |
| « A18 » | 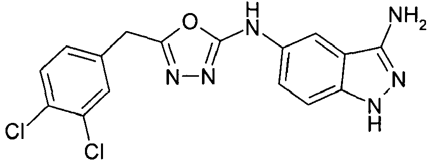 |
| | N5-[5-(3,4-Dichlorobenzyl)-[1,3,4]oxadiazol-2-yl]-1*H-*indazole-3,5-diamine |
| « A19 » | 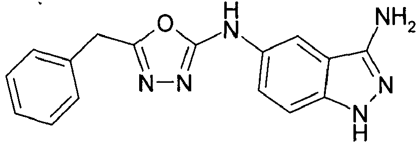 |
| | N5-(5-Benzyl-[1,3,4]oxadiazol-2-yl)-1*H*-indazole-3,5-diamine |
| « A20 » | 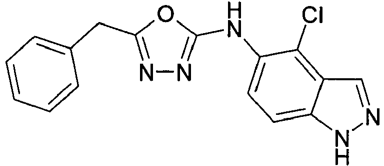 |
| | (5-Benzyl-[1,3,4]oxadiazol-2-yl)-(4-chloro-1*H*-indazol-5-yl)-amine |
| « A21 » | 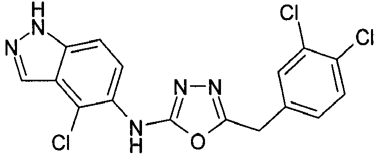 |
| | (4-Chloro-1*H*-indazol-5-yl)-[5-(3,4-dichlorobenzyl)-[1,3,4]oxadiazol-2-yl]amine |
| « A22 » | 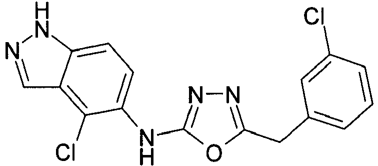 |
| | [5-(3-Chlorobenzyl)-[1,3,4]oxadiazol-2-yl]-(4-chloro-1*H-*indazol-5-yl)amine |
| « A23 » | 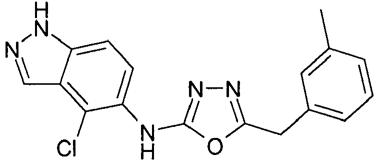 |
| | (4-Chloro-1*H*-indazol-5-yl)-[5-(3-méthylbenzyl)-[1,3,4]oxadiazol-2-yl]amine |
| « A24 » | 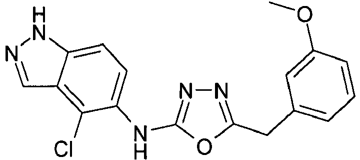 |
| | (4-Chloro-1*H*-indazol-5-yl)-[5-(3-méthoxybenzyl)-[1,3,4]oxadiazol-2-yl]amine |
| « A25 » | 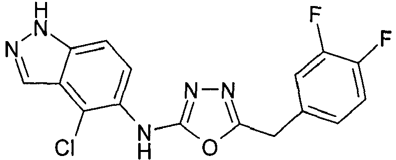 |
| | (4-Chloro-1*H*-indazol-5-yl)-[5-(3,4-difluorobenzyl)-[1,3,4]oxadiazol-2-yl]amine |
| « A26 » | 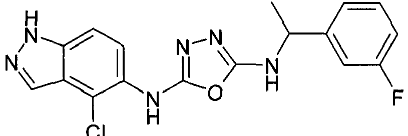 |
| | N-(4-Chloro-1*H*-indazol-5-yl)-N'-[1-(3-fluorophényl)éthyl]-[1,3,4]oxadiazole-2,5-diamine |
| « A27 » | 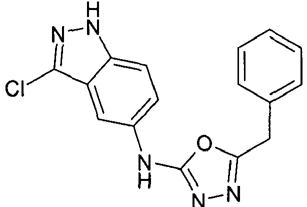 |
| | (5-Benzyl-[1,3,4]oxadiazol-2-yl)-(3-chloro-1*H*-indazol-5-yl)amine |
| « A28 » | 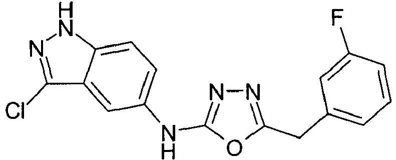 |
| | (3-Chloro-1*H*-indazol-5-yl)-[5-(3-fluorobenzyl)-[1,3,4]oxadiazol-2-yl]amine |
| « A29 » | 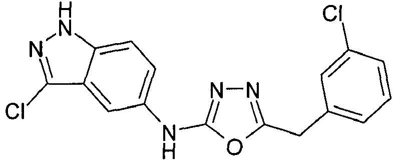 |
| | [5-(3-Chlorobenzyl)-[1,3,4]oxadiazol-2-yl]-(3-chloro-1*H-*indazol-5-yl)amine |
| « A30 » | 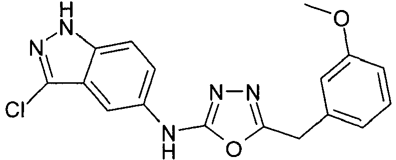 |
| | (3-Chloro-1*H*-indazol-5-yl)-[5-(3-méthoxybenzyl)-[1,3,4]oxadiazol-2-yl]amine |
| « A31 » | 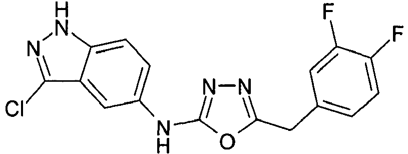 |
| | (3-Chloro-1*H*-indazol-5-yl)-[5-(3,4-difluorobenzyl)-[1,3,4]oxadiazol-2-yl]amine |
| « A32 » | 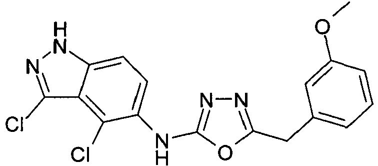 |
| | (3,4-Dichloro-1*H*-indazol-5-yl)-[5-(3-méthoxybenzyl)-[1,3,4]oxadiazol-2-yl]amine |
| « A33 » | 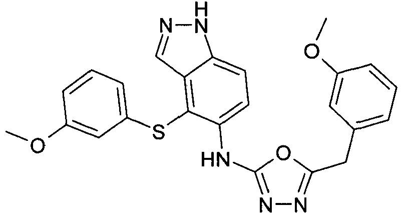 |
| | [5-(3-Méthoxybenzyl)-[1,3,4]oxadiazol-2-yl]-[4-(3-méthoxyphénylsulfanyl)-1*H*-indazol-5-yl]amine |
| « A34 » | 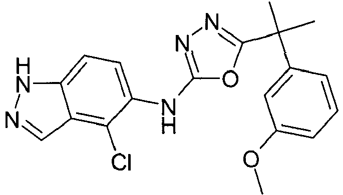 |
| | (4-Chloro-1*H*-indazol-5-yl)-{5-[1-(3-méthoxyphényl)-1-méthyléthyl]-[1,3,4]oxadiazol-2-yl}amine |
| « A35 » | 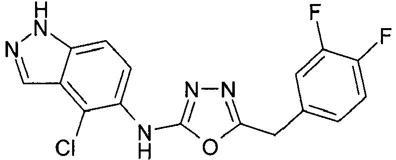 |
| | (4-Chloro-1*H*-indazol-5-yl)-[5-(3,4-difluorobenzyl)-[1,3,4]oxadiazol-2-yl]amine |
| « A36 » | 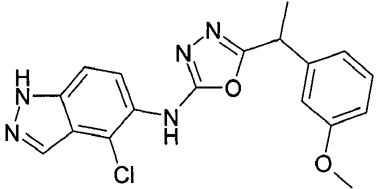 |
| | (4-Chloro-1*H*-indazol-5-yl)-{5-[1-(3-méthoxyphényl)éthyl]-[1,3,4]oxadiazol-2-yl}amine |
| « A37 » | 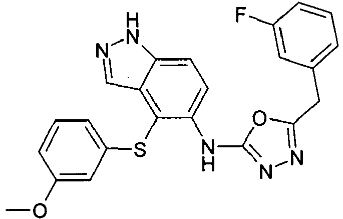 |
| | [5-(3-Fluorobenzyl)-[1,3,4]oxadiazol-2-yl]-[4-(3-méthoxyphénylsulfanyl)-1*H*-indazol-5-yl]amine |
| « A38 » | 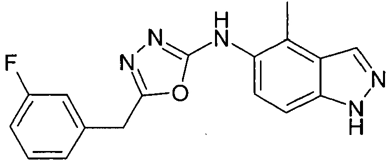 |
| | [5-(3-Fluorobenzyl)-[1,3,4]oxadiazol-2-yl]-(4-méthyl-1*H-*indazol-5-yl)amine |
| « A39 » | 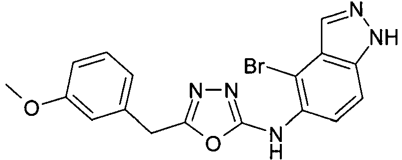 |
| | (4-Bromo-1*H*-indazol-5-yl)-[5-(3-méthoxybenzyl)-[1,3,4]oxadiazol-2-yl]amine |
| « A40 » | 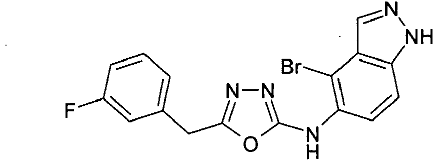 |
| | (4-Bromo-1*H*-indazol-5-yl)-[5-(3-fluorobenzyl)-[1,3,4]oxadiazol-2-yl]amine |
| « A41 » | 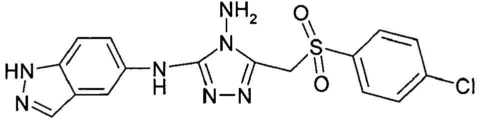 |
| | 5-(4-Chlorobenzènesulfonylméthyl)-N3-(1*H*-indazol-5-yl)-[1,2,4]triazole-3,4-diamine |
| « A42 » | 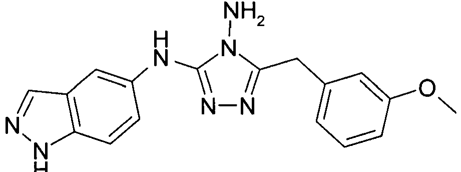 |
| | N3-(1*H*-Indazol-5-yl)-5-(3-méthoxybenzyl)-[1,2,4]triazole-3,4-diamine |
| «A43» | 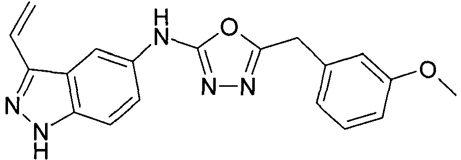 |
| | [5-(3-Méthoxybenzyl)-[1,3,4]oxadiazol-2-yl]-(3-vinyl-1*H-*indazol-5-yl)amine |
| « A44 » | 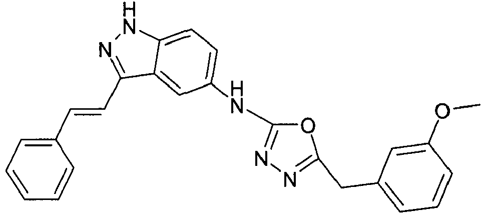 |
| | [5-(3-Méthoxybenzyl)-[1,3,4]oxadiazol-2-yl]-[3-((E)-styryl)-1*H*-indazol-5-yl]amine |
| « A45 » | 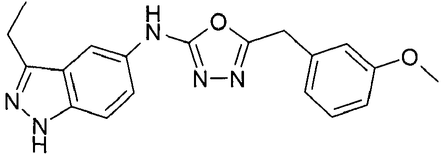 |
| | (3-Éthyl-1*H*-indazol-5-yl)-[5-(3-méthoxybenzyl)-[1,3,4]oxadiazol-2-yl]amine |
et les sels, solvats et stéréoisomères pharmaceutiquement utilisables de ceux-ci, y compris des mélanges de ceux-ci en toutes proportions.

2. Médicaments comprenant au moins un composé selon la revendication 1 et/ou des sels, solvats et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1, et de sels, solvats et stéréoisomères pharmaceutiquement utilisables de ceux-ci, y compris des mélanges de ceux-ci en toutes proportions, pour la préparation d'un médicament destiné au traitement ou à la prévention du diabète, de l'obésité, du syndrome métabolique (dyslipidémie), de l'hypertension systémique et pulmonaire, des maladies cardiovasculaires et des maladies rénales, généralement dans tous les types de fibroses et de processus inflammatoires, du cancer, des cellules tumorales, des métastases tumorales, des coagulopathies, de l'excitabilité neuronale, du glaucome, des cataractes, des infections bactériennes et pour la thérapie anti-infectieuse, pour augmenter les capacités d'apprentissage et l'attention, et pour le traitement et la prophylaxie du stress et du vieillissement cellulaires, et pour le traitement de l'acouphène.

4. Utilisation selon la revendication 3, dans laquelle le diabète est le diabète sucré, la néphropathie diabétique, la neuropathie diabétique, l'angiopathie diabétique et la microangiopathie.

5. Utilisation selon la revendication 3, dans laquelle les maladies cardiovasculaires sont les fibroses cardiaques après un infarctus du myocarde, l'hypertrophie cardiaque, l'insuffisance cardiaque et l'artériosclérose.

6. Utilisation selon la revendication 3, dans laquelle les maladies rénales sont la glomérulosclérose, la néphrosclérose, la néphrite, la néphropathie et l'altération de l'excrétion des électrolytes.

7. Utilisation selon la revendication 3, dans laquelle les fibroses et les processus inflammatoires sont la cirrhose hépatique, la fibrose pulmonaire, la pancréatite fibrosante, les rhumatismes et les arthroses, la maladie de Crohn, la bronchite chronique, la fibrose induite par une radiothérapie, la sclérodermie, la mucoviscidose, la cicatrisation et la maladie d'Alzheimer.

8. Médicaments comprenant au moins un composé selon la revendication 1 et/ou des sels, solvats et stéréoisomères pharmaceutiquement utilisables de celui-ci, y compris des mélanges de ceux-ci en toutes proportions, et au moins un autre composé actif médicamenteux.

9. Ensemble (kit) constitué de conditionnements séparés
(a) d'une quantité efficace d'un composé selon la revendication 1 et/ou de sels, solvats et stéréoisomères pharmaceutiquement utilisables de celui-ci, y compris des mélanges de ceux-ci en toutes proportions,
et
(b) d'une quantité efficace d'un autre composé actif médicamenteux.
